# EUROPEAN PATENT APPLICATION

(11) **EP 4 603 080 A1**
(43) Date of publication of application: **20.08.2025**
(21) Application number: 24158058.8
(22) Date of filing: 16.02.2024
(51) Int. Cl.: A61K 9/00, A61K 38/22, A61K 38/26, A61K 47/34, A61P 3/04, A61P 3/10, A61P 9/00, A61P 25/28

(54) **COMPOSITION COMPRISING SEMAGLUTIDE, CAGRILINTIDE AND A CO-POLYAMINO ACID BEARING CARBOXYLATE CHARGES AND HYDROPHOBIC RADICALS**

(71) Applicant: Adocia, 69003 Lyon (FR)
(72) Inventor: CHAN, You Ping, 69003 Lyon (FR)
(74) Representative: Casalonga

(57) **Abstract**

The invention relates to a composition comprising semaglutide, cagrilintide and a co-polyamino acid (PLG) bearing carboxylate charges and hydrophobic radicals, said composition being in the form of aqueous injectable solution at pH between 5.0 to 6.0.

## Description

The invention relates to a composition comprising semaglutide, cagrilintide and a co-polyamino acid (PLG) bearing carboxylate charges and hydrophobic radicals, said composition being in the form of aqueous injectable solution at pH between 5.0 to 6.0.

This composition may be used as a medicament, in particular for the treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

Formulating in the same composition semaglutide and cagrilintide was not considered possible, due to different physicochemical properties of these active pharmaceutical ingredients (APIs) until WO2123/187067. This application from Novo Nordisk proposes a solution to co-formulate said APIs by using a cyclodextrin comprising hydroxypropyl substitution.

The compositions of WO2023/187067 prior art needs high concentration of cyclodextrins to solubilize semaglutide. Furthermore, compositions of the prior art are not sufficiently stable, in particular regarding the level of impurities.

A goal of the invention is to allow an improved stability of of semaglutide and cagrilintide in a composition having pH between 5.0 to 6.0.

Therefore, there is a need to find a solution for obtaining stable compositions comprising semaglutide and cagrilintide.

Specifically, one of the goals of the present invention is to enhance the chemical stability of cagrilintide while having a good stability of semaglutide.

Among the problems to be solved can also be cited the improvement of local tolerance and comfort upon injection.

Another problem to be solved is to use less amount of the compound allowing the co-formulation.

Therefore, there is a need to find a solution for solving at least in part the problems cited above.

Surprisingly, the applicant has found that a composition, in the form of an aqueous injectable solution, comprising semaglutide and cagrilintide with a co-polyamino acids bearing carboxylate charges and hydrophobic radicals allows to solve at least in part the above cited problems.

The invention relates to a composition, in the form of an aqueous injectable solution, whose pH is comprised from 5.0 to 6.0, in particular from 5.1 to 5.9, comprising:
- semaglutide
- cagrilintide, and
- a co-polyamino acid (PLG) bearing carboxylate charges and hydrophobic radicals Hy, said co-polyamino acid consisting of glutamic or aspartic units, and said hydrophobic radicals Hy being according to formula X below: 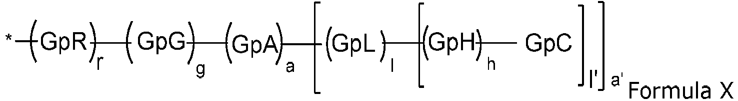 in which
- GpR is chosen from the radicals according to formulas VII, VII' or VII': 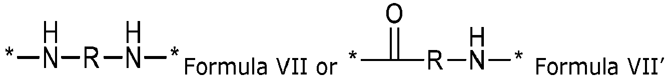 or 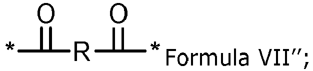 identical or different GpG and GpH are chosen from the radicals according to formulas XI or XI': 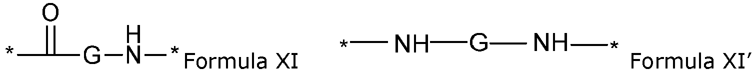
- GpA is chosen from the radicals according to formula VIII: 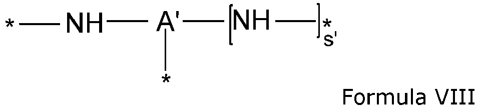
   _{∘} in which A' is chosen from the radicals according to formula VIII', VIII" or VIII‴: 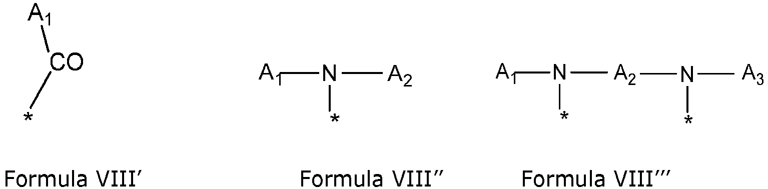
   - GpL is chosen from the radicals according to formula XII: 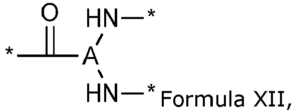
   - GpC is a radical according to formula IX: 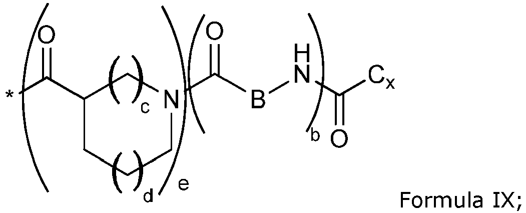 the * indicates the attachment sites of the different groups bonded by the amide functions;
   - a is an integer equal to 0 or to 1 and a' = 1 if a = 0 and a' = 1, 2 or 3 if a = 1;
   - a' is an integer equal to 1, to 2, or to 3
   - b is an integer equal to 0 or to 1;
   - c is an integer equal to 0 or to 1, and if c is equal to 0, then d is equal to 1 or to 2;
   - d is an integer equal to 0, to 1, or to 2;
   - e is an integer equal to 0 or to 1;
   - g is an integer equal to 0, to 1, to 2, to 3, to 4, to 5 or to 6;
   - h is an integer equal to 0, to 1, to 2, to 3, to 4, to 5 or to 6;
   - I is an integer equal to 0 or 1 and I' = 1 if I = 0, and I' = 2 if I = 1;
   - r is an integer equal to 0, to 1, or to 2, and
   - s' is an integer equal to 0 or 1, and
   - if e is different from 0, then at least one of g, h or I is different from 0;
   - A, A₁, A₂ and A₃, identical or different, are linear or branched alkyl radicals, and/or substituted by a radical derived from a saturated, unsaturated or aromatic ring, comprising 1 to 8 carbon atoms,
   - B is a linear or branched alkyl radical and/or comprising an aromatic nucleus, comprising 1 to 9 carbon atoms or an unsubstituted ether or polyether radical comprising from 4 to 14 carbon atoms and from 1 to 5 oxygen atoms;
   - Cₓ is a linear or branched monovalent alkyl radical, and/or comprising a cyclic part, in which x indicates the number of carbon atoms and:
      _{∘} when the hydrophobic radical -Hy carries 1 -GpC, then 9 ≤ x ≤ 25
      _{∘} when the hydrophobic radical -Hy carries 2 -GpC, then 9 ≤ x ≤ 15,
      _{∘} when the hydrophobic radical -Hy carries 3 -GpC, then 7 ≤ x ≤ 13,
      _{∘} when the hydrophobic radical -Hy carries 4 -GpC, then 7 ≤ x ≤ 11,
      _{∘} when the hydrophobic radical -Hy carries at least 5 -GpC, then 6 ≤ x ≤ 11,
   - G is a branched alkyl radical of 1 to 8 carbon atoms, with said alkyl radical bearing one or more free carboxylic acid functions,
   - R is a radical chosen from the group consisting of a divalent alkyl radical, linear or branched, comprising 1 to 12 carbon atoms, a divalent alkyl radical, linear or branched, comprising 1 to 12 carbon atoms bearing one or more -CONH₂ functions, or a unsubstituted ether or polyether radical comprising from 4 to 14 carbon atoms and 1 to 5 oxygen atoms:
   - the hydrophobic radical(s) -Hy according to formula X being bonded to the PLG:
      _{∘} via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
      _{∘} via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,
   - the ratio M between the number of hydrophobic radicals and the number of glutamic or aspartic units being from 0 < M ≤ 0.5;
   - when several hydrophobic radicals are borne by a co-polyamino acid, then they are identical or different,
   - the degree of polymerization DP in glutamic or aspartic units for PLG chains is comprised from 5 to 250;
   - free carboxylic acid functions being in the form of an alkali metal salt chosen from the group consisting of Na⁺ and K⁺.

The invention also concerns compositions for use as a medicament.

The invention also concerns compositions for use in a method of treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

The invention also concerns an injection device comprising a composition according to the invention.

The invention also concerns a container, such as a vial or a cartridge comprising a composition according to the invention.

The invention also concerns a method for the preparation of a stable, injectable aqueous solutions of semaglutide comprising the step of mixing a co-polyamino acid with the semaglutide.

### DEFINITIONS

By "physically stable composition" is meant compositions that meet the visual inspection criteria described in European, American and international pharmacopoeia, that is, compositions that are clear and that do not contain visible particles and are also colorless.

The "chemical stability" can be evaluated by RP-HPLC for estimating peptide purity and SEC-HPLC for estimating high molecular weight products (HMWP).

By "injectable aqueous solution" is meant solutions for which the solvent is water and which meets the pharmacopoeia conditions of Europe and the US.

Compositions in the form of an injectable aqueous solution according to the invention are clear solutions. By "clear solution" is meant compositions that meet the criteria described in American and European pharmacopoeia regarding injectable solutions. In US pharmacopoeia, solutions are defined in part <1151>, referring to injection <1> (referring to <788> according to USP 35 and specified in <788> according to USP 35 and in <787>, <788> and <790> USP 38 (beginning on August 1st, 2014), according to USP 38). In European pharmacopoeia, injectable solutions must meet the criteria provided in sections 2.9.19 and 2.9.20.

By "co-polyamino acid being constituted of glutamic or aspartic units" is meant linear, non-cyclic units of glutamic or aspartic acids bonded to each other by peptidic bonds, said chains presenting a C-terminal part, corresponding to carboxylic acid at one end, and an N-terminal part, corresponding to amine at the other end of the chain. The N-terminal part and the C-terminal part may be substituted, in particular as defined in Formulae XXXa, XXXa', XXXb and XXXb'.

In the following, and without other indication, the limits of a value range are included within this range, particularly in the expressions "between" and "ranging from ... to ...".

### Co-polyamino acid

Said co-polyamino acid bearing carboxylate charges and hydrophobic -Hy radicals is soluble in an aqueous solution of pH between 5.0 and 6.0, at a temperature of 25 °C and at a concentration of 50 mg/ml.

Said co-polyamino acid bearing carboxylate charges and hydrophobic -Hy radicals is soluble in an aqueous solution of pH between 5.0 and 6.0, at a temperature of 25 °C and at a concentration of 20 mg/ml.

The co-polyamino acid is a random co-polyamino acid in the chain of glutamic and/or aspartic units.

By "alkyl radical" is meant a carbon-comprising chain, linear or branched, which does not include a heteroatom.

In the formulas, the *s indicate the attachment sites of the various elements represented.

The radicals Hy, GpR, GpG, GpH, GpA, GpL and GpC are each independently identical or different from one residue to another.

In one embodiment, the composition according to the invention is characterized in that the Hy comprises between 15 and 100 atoms of carbon.

In one embodiment, the composition according to the invention is characterized in that the Hy comprises between 30 and 70 atoms of carbon.

In one embodiment, the composition according to the invention is characterized in that the Hy comprises between 40 and 60 atoms of carbon.

In one embodiment, the composition according to the invention is characterized in that the Hy comprises between 20 and 30 atoms of carbon.

In one embodiment, the composition according to the invention is characterized in that the Hy comprises more than 30 atoms of carbon.

In the formulas, the *s indicate the attachment sites of hydrophobic radicals to the PLG or between the different GpR, GpG, GpH, GpA, GpL and GpC to form amide functions.

Hy radicals are attached to the PLG via amide functions.

In one embodiment, r=0 and the hydrophobic radical of formula X is bonded to the PLG via a covalent bond between a carbonyl of the hydrophobic radical and a nitrogen atom borne by the PLG, thus forming an amide function resulting from the reaction of an amine function borne by the PLG precursor and and acid function borne by the -Hy' precursor of the hydrophobic radical.

In one embodiment, r=1 or 2 and the hydrophobic radical of formula X is bonded to the PLG:
▪ via a covalent bond between a nitrogen atom of the hydrophobic radical and a carbonyl borne by the PLG, thus forming an amide function resulting from the reaction of an amine function of the -Hy' precursor of the hydrophobic radical and an acid function borne by the PLG,
   or
▪ via a covalent bond between a carbonyl of the hydrophobic radical and a nitrogen atom borne by the PLG, thus forming an amide function resulting from the reaction of an amine function of the Hy' precursor of the hydrophobic -Hy radical and an acid function borne by the PLG.

In one embodiment, if GpA is a radical of formula VIIIc and r=1, then:
- the GpCs are directly or indirectly bonded to N_{α1} and N_{α2} and the PLG is directly or indirectly bonded via GpR to N_{β1} , or
- the GpCs are directly or indirectly bonded to N_{α1} and N_{β1} and the PLG is directly or indirectly bonded via GpR to N_{α2} , or
- the GpCs are directly or indirectly bonded to N_{α2} and N_{β1} and the PLG is directly or indirectly bonded via GpR to N_{α1}.

In one embodiment, if GpA is a radical of formula VIIIc and r=0, then:
- the GpCs are directly or indirectly bonded to N_{α1} and N_{α2} and the PLG is directly or indirectly bonded to N_{β1}; or
- the GpCs are directly or indirectly bonded to N_{α1} and N_{β1} and the PLG is directly or indirectly bonded to N_{α2}; or
- the GpCs are directly or indirectly bonded to N_{α2} and N_{β1}, and the PLG is directly or indirectly bonded to N_{α1}.

In one embodiment, if GpA is a radical of formula VIIId and r=1, then
- the GpCs are directly or indirectly bonded to N_{α1}, N_{α2} and N_{β1} and the PLG is directly or indirectly bonded via GpR to N_{β2}, or
- the GpCs are directly or indirectly bonded to N_{α1}, N_{α2} and N_{β2} and the PLG is directly or indirectly bonded via GpR to N_{β1}, or
- the GpCs are directly or indirectly bonded to N_{α1}, N_{β1} and N_{β2} and the PLG is directly or indirectly bonded via GpR to N_{α2}, or
- the GpCs are directly or indirectly bonded to N_{α2}, N_{β1} and N_{β2} and the PLG is directly or indirectly bonded via GpR to N_{α1}.

In one embodiment, if GpA is a radical of formula VIIId and r=0, then
- the GpCs are directly or indirectly bonded to N_{α1}, N_{α2} and N_{β1} and the PLG is directly or indirectly bonded to N_{β2}; or
- the GpCs are directly or indirectly bonded to N_{α1}, N_{α2} and N_{β2} and the PLG is directly or indirectly bonded to N_{β1}; or
- the GpCs are directly or indirectly bonded to N_{α1}, N_{β1} and N_{β2} and the PLG is directly or indirectly bonded to N_{α2}; or
- the GpCs are directly or indirectly bonded to N_{α2}, N_{β1} and N_{β2} and the PLG is directly or indirectly bonded to N_{α1.*}.

In one embodiment, when r=2, then the GpR group bonded to the PLG is chosen from among the GpRs of formula VII.

In one embodiment, when r=2, then the GpR group bonded to the PLG is chosen from among the Gprs of formula VII and the second GpR is chosen from among the GpRs of formula VII".

In one embodiment, an embodiment, when r=2 then the GpR bonded to the PLG is chosen from among the GpRs of formula VII".

In one embodiment, an embodiment, when r=2, then the GpR group bonded to the PLG is chosen from among the Gprs of formula VII" and the second GpR is chosen from among the GpRs of formula VII.
In one embodiment, a=0,
In one embodiment, h=1 and g=0,
In one embodiment, h=0 and g=1,
In one embodiment, r=0, g=1 and h=0.

In one embodiment, at least one of g, h or I is different from 0.

In one embodiment, at least one of g and h is equal to 1.

In one embodiment, a=1 and I=1.

In one embodiment, if I=0, at least one of g or h is equal to 0.

In one embodiment, if I=1, at least one of g or h is equal to 0.

In one embodiment, g+h≥2.

In one embodiment, g is greater than or equal to 2 (g≥2).

In one embodiment, h is greater than or equal to 2 (h≥2).

In one embodiment, g+h≥2 and a and I are equal to 0 (a=I=0).

In one embodiment, g+h≥2 and b is equal to 0 (b=0).

In one embodiment, g is greater than or equal to 2 (g≥2) and b is equal to 0.

In one embodiment, g+h≥2, b is equal to 0 (b=0) and e is equal to 1 (e=1).

In one embodiment, g or h is greater than or equal to 2 (g≥2) b is equal to 0 (b=0) and e is equal to 1 (e=1).

In one embodiment, said at least one hydrophobic radical -Hy is chosen from among the radicals of formula X in which r=2 of formula Xc', as defined below: 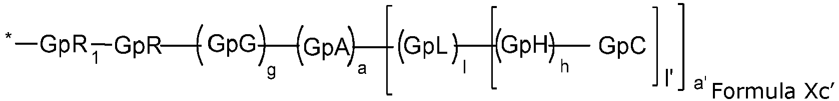 in which GpR₁ is a radical from formula VII. 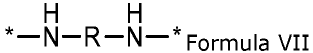 in which GpR, GpG, GpA, GpL, GpH, GpC, R, a, a', g, h, I and I' have the definitions given above.

In one embodiment, said at least one hydrophobic radical -Hy is chosen from among the radicals of formula X in which r=2 of formula Xc', as defined below: 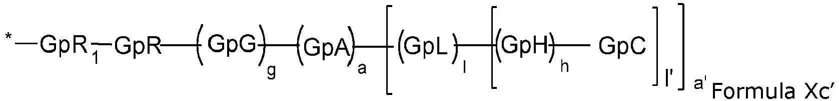 in which GpR₁, is a radical from formula VII". 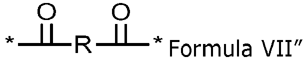 in which GpR, GpG, GpA, GpL, GpH, GpC, R, a, a', g, h, I and I' have the definitions given above.

In one embodiment, g=h=0, a=1, GpA is a radical from Formula VIII with s'=1 and A' from Formula VIII' or VIII", and I=1.

In one embodiment, said at least one hydrophobic radical -Hy is chosen from among the radicals of formula X in which r=2 of formula Xc', as defined below: 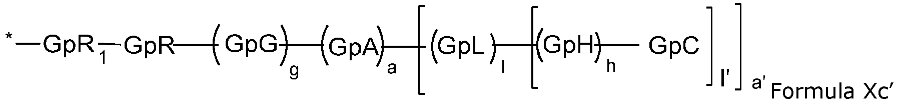 in which GpR₁ is a radical from formula VII. 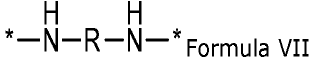 in which GpR, GpG, GpA, GpL, GpH, GpC, R, a, a', g, h, I and I' have the definitions given above.

In one embodiment, said at least one hydrophobic radical -Hy is chosen from among the radicals of formula X in which r=2 of formula Xc', as defined below: 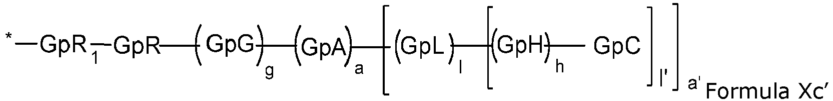 in which GpR₁, is a radical from formula VII". 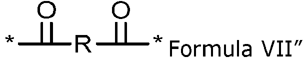 in which GpR, GpG, GpA, GpL, GpH, GpC, R, a, a', g, h, I and I' have the definitions given above.

In one embodiment, said at least one hydrophobic radical -Hy is chosen from among the radicals of formula X in which
- I=0,
- from formula Xb' as defined below. 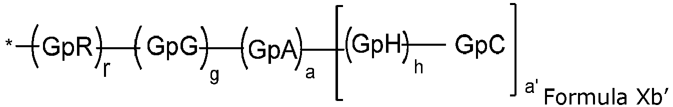 in which
- GpR is chosen from among the radicals of formulas VII, VII' or VII": 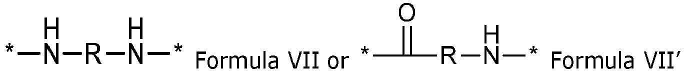 or 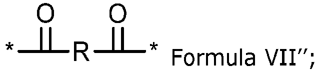
- GpG is chosen from among the radicals of formula XI or XI': 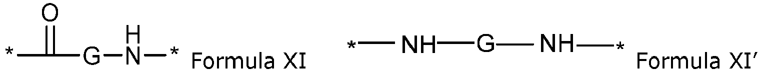
- GpA is chosen from among the radicals of formula VIII in which s'=1 represented by formula VIIIa or formula VIII in which and [sic] s'=0 represented by formula VIIIb. 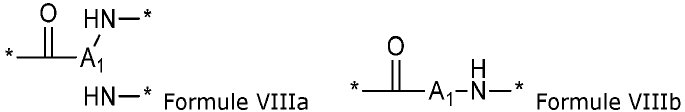
- GpC is a radical from formula IX: 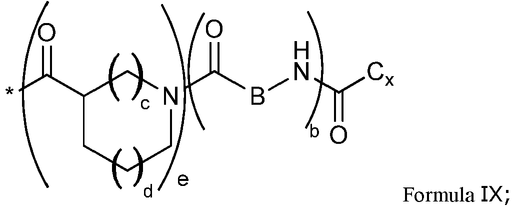
- The *s indicate the attachment sites of the different groups bonded by the amide functions;
- a is an integer equal to 0 or to 1 and a' = 1 if a = 0'= if and a'=1 or a'=2 if a = 1;
- a' is an integer equal to 1 or 2 and
   _{∘} If a' is equal to 1 then a is equal to 0 or to 1 and GpA is a radical from formula VIIIb and,
   _{∘} if a' is equal to 2 then a is equal to 1 and GpA is a radical from formula VIIIa;
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6, and at least one of g or h is different from 0;
- r is an integer equal to 0, to 1, or to 2, and
- s' is a whole number equal to 0 or 1;
- A₁ is a linear or branched alkyl radical, and possibly substituted by a radical from a saturated, unsaturated or aromatic ring, comprising 1 to 6 carbon atoms.
- B is a linear or branched alkyl radical possibly comprising an aromatic core, comprising 1 to 9 carbon atoms or a non-substituted ether or polyether radical comprising 4 to 14 carbon atoms and 1 to 5 oxygen atoms;
- Cₓ is a linear or branched monovalent alkyl radical, possibly comprising a ring part, in which x indicates the number of carbon atoms and:
   ▪ When the hydrophobic radical -Hy carries 1 -GpC, then 9 ≤ x ≤ 25,
   ▪ When the hydrophobic radical -Hy carries 2 -GpC, then 9 ≤ x ≤ 15,
   ▪ When the hydrophobic radical -Hy carries 3 -GpC, then 7 ≤ x ≤ 13,
   ▪ When the hydrophobic radical -Hy carries 4 -GpC, then 7 ≤ x ≤ 11,
   ▪ When the hydrophobic radical -Hy carries at least 5 -GpC, then 6 ≤ x ≤ 11,
- G is a branched alkyl radical of 1 to 8 carbon atoms, with said alkyl radical bearing one or more free carboxylic acid functions,
- R is a radical chosen from the group constituted by a divalent alkyl radical, linear or branched, comprising 1 to 12 carbon atoms, a divalent alkyl radical, linear or branched, comprising 1 to 12 carbon atoms bearing one or more - CONH₂ functions, or a non-substituted ether or polyether radical comprising from 4 to 14 carbon atoms and 1 to 5 oxygen atoms:
- The hydrophobic Hy radical(s) of formula X being bonded to the PLG:
   _{∘} via a covalent bond between a carbonyl of the hydrophobic radical and a nitrogen atom borne by the PLG, thus forming an amide function resulting from the reaction of an amine function borne by the PLG and and acid function borne by the precursor of the hydrophobic,
      or
   _{∘} via a covalent bond between a nitrogen atom of the hydrophobic radical and a carbonyl borne by the PLG. Thus forming an amide function resulting from the reaction of an amine function of the -Hy' precursor of the hydrophobic radical and an acid function borne by the PLG,
- the ratio M between the number of hydrophobic radicals and the number of glutamic or aspartic units being between 0 < M ≤ 0,5;
- when several hydrophobic radicals are borne by a co-polyamino acid, then they are identical or different,
- free carboxylic acid functions being in the form of an alkali metal salt chosen from the group constituted by Na⁺ and K⁺.

In one embodiment said at least one hydrophobic radical -Hy is chosen from among the radicals of formula X as defined below in which I= 0,
- GpA is chosen from among the radicals in formula VIII in which s'=1 and A' is chosen from among the radicals of formula VIII" or VIII"',
- from formula Xb' as defined below: 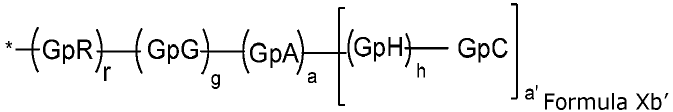 in which
- GpR is chosen from among the radicals of formulas VII, VII' or VII": 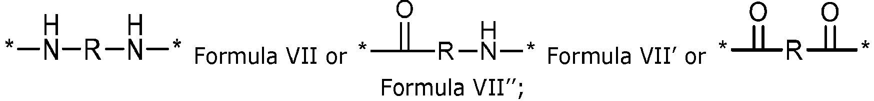
- GpG is chosen from among the radicals of formula XI or XI': 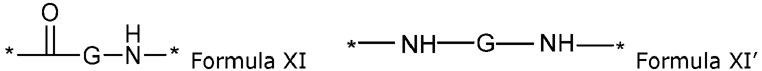
- GpA is chosen from among the radicals of formulas VIIIc or VIIId: 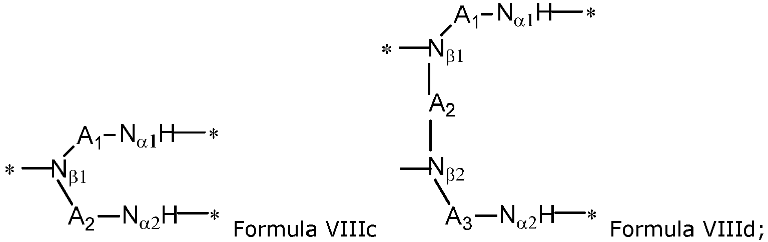
- GpC is a radical from formula IX: 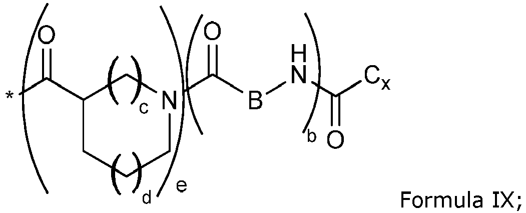
- The *s indicate the attachment sites of the different groups bonded by the amide functions;
- a is a whole equal to 0 or to 1 and a' = 1 if a = 0, and a' = 2 or 3 if a = 1;
- a' is an integer equal to 2 or 3 and
   _{∘} if a' is equal to 1 then a is equal to 0 and,
   _{∘} if a' is equal to 2 or 3 then a is equal to 1 and GpA is a radical from formula VIIIc or VIIId;
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6, and at least one of g or h is different from 0;
- r is whole number equal to 0, to 1, or to 2, and
- s' is an integer equal to 1;
- A₁, A₂, A₃ identical or different, are linear or branched alkyl radicals, and possibly substituted by a radical from a saturated, unsaturated or aromatic ring, comprising 1 to 6 carbon atoms.
- B is a linear or branched alkyl radical possibly comprising an aromatic core, comprising 1 to 9 carbon atoms or a non-substituted ether or polyether radical comprising 4 to 14 carbon atoms and 1 to 5 oxygen atoms;
- Cₓ is a linear or branched monovalent alkyl radical, possibly comprising a ring part, in which x indicates the number of carbon atoms and:
   ▪ When the hydrophobic radical -Hy carries 1 -GpC, then 9 ≤ x ≤ 25,
   ▪ When the hydrophobic radical -Hy carries 2 -GpC, then 9 ≤ x ≤ 15,
   ▪ When the hydrophobic radical -Hy carries 3 -GpC, then 7 ≤ x ≤ 13,
   ▪ When the hydrophobic radical -Hy carries 4 -GpC, then 7 ≤ x ≤ 11,
   ▪ When the hydrophobic radical -Hy carries 5 -GpC, then 6 ≤ x ≤ 11,
- The hydrophobic Hy radical(s) of formula X being bonded to the PLG:
   _{∘} via a covalent bond between a carbonyl of the hydrophobic radical and a nitrogen atom borne by the PLG, thus forming an amide function resulting from the reaction of an amine function borne by the PLG and and acid function borne by the -Hy' precursor of the hydrophobic radical,
      or
   _{∘} via a covalent bond between a nitrogen atom of the hydrophobic radical and a carbonyl borne by the PLG. Thus forming an amide function resulting from the reaction of an amine function of the -Hy' precursor of the hydrophobic radical and an acid function borne by the PLG,
- G is a branched alkyl radical of 1 to 8 carbon atoms, with said alkyl radical bearing one or more free carboxylic acid functions,
- R is a radical chosen from the group constituted by a divalent alkyl radical, linear or branched, comprising 1 to 12 carbon atoms, a divalent alkyl radical, linear or branched, comprising 1 to 12 carbon atoms bearing one or more -CONH₂ functions, or a non-substituted ether or polyether radical comprising from 4 to 14 carbon atoms and 1 to 5 oxygen atoms:
- the ratio M between the number of hydrophobic radicals and the number of glutamic or aspartic units being between 0 < M ≤ 0.5;
- when several hydrophobic radicals are borne by a co-polyamino acid, then they are identical or different,
- free carboxylic acid functions being in the form of an alkali metal salt chosen from the group constituted by Na⁺ and K⁺.

In one embodiment, said at least one hydrophobic radical -Hy is chosen from among the radicals of formula X in which a=1 and a'=1 of formula Xa, as defined below: 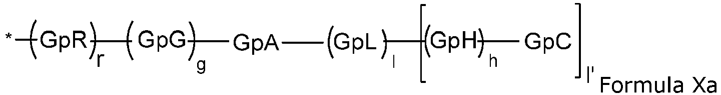 in which GpA is a radical of formula VIII and A' is chosen from among the radicals of formula VIII' with s'=0 and GpA is a radical of Formula VIIIb 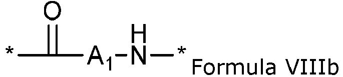 and GpR, GpG, GpL, GpH, GpC, A₁, r, g, h, I and I' have the definitions given above.

In one embodiment, said at least one hydrophobic radical -Hy is chosen from among the radicals of formula X in which a=1 of formula Xb, as defined below: 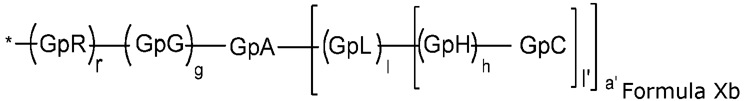 in which GpA is a radical of formula VIII and A' is chosen from among the radicals of formula VIII' with s'=1 and GpA is a radical of Formula VIIIa 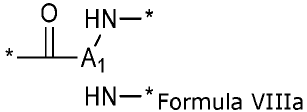 and GpR, GpG, GpL, GpH, GpC, A₁, a', r, g, h, I and I' have the definitions given above.

In one embodiment, said at least one hydrophobic radical -Hy is chosen from among the radicals of formula X in which a=1, as defined below: 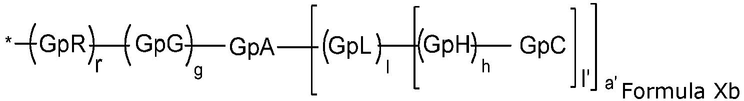 in which GpA is a radical from formula VIII and A is chosen from among the radicals of Formula VIII" with s'=1 and GpA is a radical of Formula VIIIc 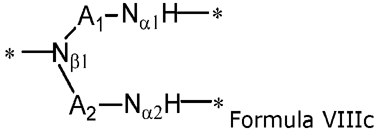 and GpR, GpG, GpL, GpH, GpC, A₁, A₂, r, g, h, a', I and I' have the definitions given above.

In one embodiment, said at least one hydrophobic radical -Hy is chosen from among the radicals of formula X in which a=1, as defined below: 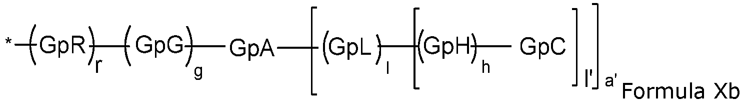 in which GpA is a radical of formula VIII and A is chosen from among the radicals of formula VIII‴ with s'=1, and GpA is a radical of Formula VIIId 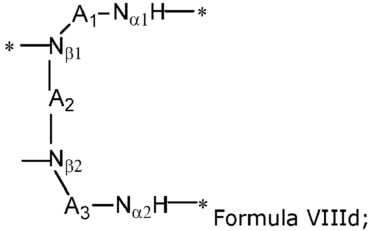 and GpR, GpG, GpL, GpH, GpC, A₁, A₂, A₃, a', r, g, h, I and I' have the definitions given above.

In one embodiment, the composition according to the invention is characterized in that said hydrophobic radicals are chosen from among the hydrophobic radicals of formula X in which GpA is a radical from formula VIIIb, a'=1 and I=0, represented by the following formula Xe: 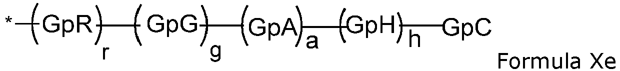 GpR, GpG, GpA, GpH, GpC, r, g, h, and a have the definitions given above.

In one embodiment, r=0, and GpA is chosen from among the radicals of formulas VIIIa and VIIIb.

In one embodiment, r=0, g=0 and GpA is chosen from among the radicals of formula VIIIa and VIIIb.

In one embodiment, r=0, and GpA is chosen from among the radicals of formulas VIIIa and VIIIb and h=0.

In one embodiment, said at least one hydrophobic radical -Hy is chosen from among the radicals of formula X in which r=1 of formula Xc, as defined below: 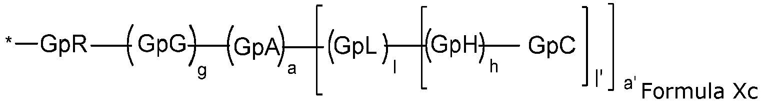 in which GpR is a radical from formula VII. 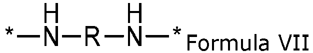 and GpG, GpA, GpL, GpH, GpC, R, a, a', g, h, I, a' and I' have the definitions given above.

In one embodiment, said at least one hydrophobic radical -Hy is chosen from among the radicals of formula X in which r=1 of formula Xc, as defined below: 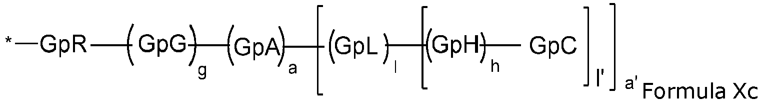 in which GpR is a radical from formula VII'. 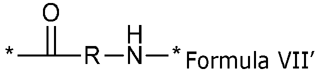 and GpG, GpA, GpL, GpH, GpC, R, a, a', g, h, I, a' and I' have the definitions given above.

In one embodiment, said at least one hydrophobic radical -Hy is chosen from among the radicals of formula X in which r=1 of formula Xc, as defined below: 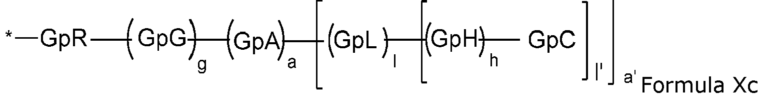 in which GpR is a radical from formula VII"

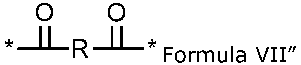

In one embodiment, r=1, and GpR is chosen from among the radicals of formulas VII' and VII" and h=0.

In one embodiment, r = 1, g = 0 and GpR is a radical of formula VII' and h=0.

In one embodiment, r =1, g = 0 and GpR is a radical of formula VII' and h=1.

In one embodiment, r=1, g = 0 and GpR is a radical of formula VII', GpA is chosen from among the radicals of Formula VIIIa or VIIIb and h=0.

In one embodiment, r=1, g = 0, GpR is a radical of formulas =VII', GpA is chosen from among the radicals of formula VIIIa or VIIIb and h = 1.

In one embodiment, r = 1, g = 0 GpR is a radical of formula VII', GpA is a radical from formula VIIIa and h=0.

In one embodiment, r =1, g = 0 GpR is a radical from formula VII', GpA is a radical of formula VIIIa and h=1.

In one embodiment, r = 1, g = 0, GpR is a radical from formula VII', GpA is a radical of formula VIIIb and h=0.

In one embodiment, r=1, g = 0, GpR is a radical of formulas =VII', GpA is a radical of formula VIIIb and h = 1.

In one embodiment, said at least one hydrophobic radical -Hy is chosen from among the radicals of formula X, as defined below: 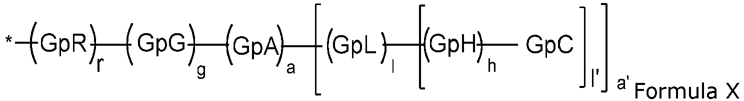 in which GpC is a radical from formula IX in which e = 0 and GpC is a radical from formula IXa.

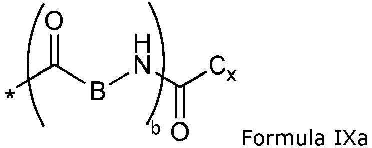

In one embodiment, said at least one hydrophobic radical -Hy is chosen from among the radicals of formula X, as defined below: 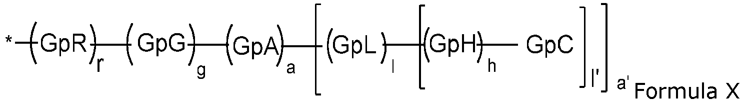 in which GpC is a radical from formula IX in which e = 1, b = 0 and GpC is a radical from formula IXd.

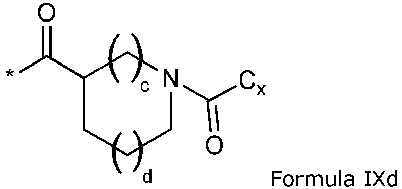

In one embodiment, said at least one hydrophobic radical -Hy is chosen from among the radicals of formula X, as defined below: 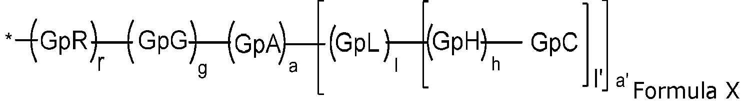 in which GpC is a radical from formula IX in which e = 1 and GpC is a radical from formula IXb

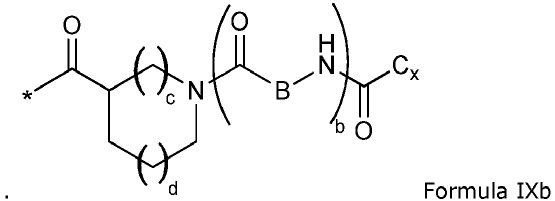

In one embodiment, said at least one hydrophobic radical -Hy is chosen from among the radicals of formula X in which r, g, a, I, h are equal to 0 of formula Xd, as defined below:

*-GpC Formula Xd,

in which GpC is a radical from formula IX in which e = 0, b = 0 and GpC is a radical from formula IXc.

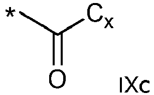

In one embodiment, the composition according to the invention is characterized in that said hydrophobic radicals are chosen from among the hydrophobic radicals of formula X in which a'=2 and a = 1 and I=0, represented by the following formula Xf: 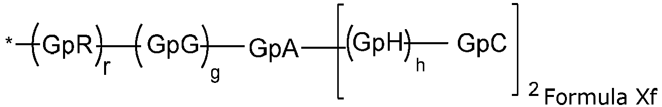 GpR, GpG, GpA, GpH, GpC, r, g and h have the definitions given above.

In one embodiment, the composition according to the invention is characterized in that said hydrophobic radicals are chosen from among the hydrophobic radicals of formula X in which h=0, I=0 and I'=1, represented by the following formula Xg: 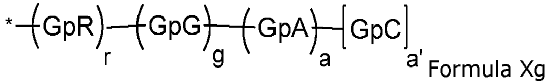 GpR, GpG, GpA, GpC, r, g, a, and a' have the definitions given above.

In one embodiment, the composition according to the invention is characterized in that said hydrophobic radicals are chosen from among the hydrophobic radicals of formula X in which h=0, a' = 1 represented by the following formula Xh: 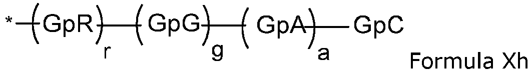 GpR, GpG, GpA, GpC, r, a and g have the definitions given above.

In one embodiment, the composition according to the invention is characterized in that said hydrophobic radicals are chosen from among the hydrophobic radicals of formula X in which h = 0, a' = 2 and a = 1, represented by the following formula Xi: 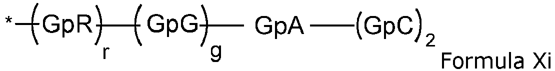 GpR, GpG, GpA, GpC, r and g have the definitions given above.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 12 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 4 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 4 atoms of carbon.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 2 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 1 to 11 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 1 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 2 to 5 atoms of carbon and bearing one or more amide functions (-CONH2).

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 5 atoms of carbon and bearing one or more amide functions (-CONH2).

In one embodiment, the composition is characterized in that the hydrophobic radical from formulas X, Xa, Xb, Xb', Xc, Xe, Xf, Xg, Xh and Xi is a radical in which R is chosen from the group constituted by the radicals represented by the formulas below:

| | |
|---|---|
| 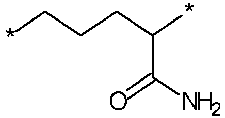 | Formula X1 |
| 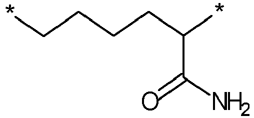 | Formula X2 |

In one embodiment, the composition is characterized in that the hydrophobic radical from formulas X, Xa, Xb, Xb', Xc, Xe, Xf, Xg, Xh and Xi is a radical in which R is a radical from Formula X1.

In one embodiment, the composition is characterized in that the hydrophobic radical from formulas X, Xa, Xb, Xb', Xc, Xe, Xf, Xg, Xh and Xi is a radical in which R is a radical from Formula X2.

In one embodiment, the composition is characterized in that the hydrophobic radical from formulas X, Xa, Xb, Xb', Xc, Xe, Xf, Xg, Xh and Xi is a radical in which R is bonded to the co-polyamino acid via an amide function borne by the carbon in delta or epsilon position (or in position 4 or 5) in relation to the amide function (-CONH₂).

In one embodiment, the composition is characterized in that the hydrophobic radical from formulas X, Xa, Xb, Xb', Xc, Xe, Xf, Xg, Xh and Xi is a radical in which R is an unsubstituted linear ether or polyether radical comprising 4 to 14 carbon atoms and from 1 to 5 oxygen atoms.

In one embodiment, the composition is characterized in that the hydrophobic radical from formulas X, Xa, Xb, Xb', Xc, Xe, Xf, Xg, Xh and Xi is a radical in which R is an ether radical.

In one embodiment, the composition is characterized in that the hydrophobic radical from formulas X, Xa, Xb, Xb', Xc, Xe, Xf, Xg, Xh and Xi is a radical in which R is an ether radical comprising 4 to 6 carbon atoms.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 6 atoms of carbon.

In one embodiment, the composition is characterized in that the hydrophobic radical from formulas X, Xa, Xb, Xb', Xc, Xe, Xf, Xg, Xh and Xi is a radical in which R is an ether radical represented by formula

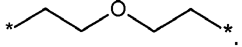

In one embodiment, the composition is characterized in that the hydrophobic radical from formulas X, Xa, Xb, Xb', Xc, Xe, Xf, Xg, Xh and Xi is a radical in which R is a polyether radical.

In one embodiment, the composition is characterized in that the hydrophobic radical from formulas X, Xa, Xb, Xb', Xc, Xe, Xf, Xg, Xh and Xi is a radical in which R is a linear polyether or polyether radical comprising 6 to 10 carbon atoms and from 2 to 3 oxygen atoms.

In one embodiment, the composition is characterized in that the hydrophobic radical from formulas X, Xa, Xb, Xb', Xc, Xe, Xf, Xg, Xh and Xi is a radical in which R is a polyether radical chosen from the group constituted by the radicals represented by the formulas below:

| | |
|---|---|
| 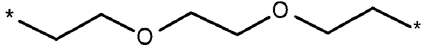 | Formula X3 |
| 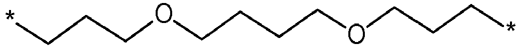 | Formula X4 |
| 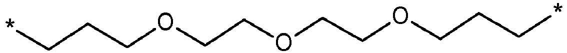 | Formula X5 |
| 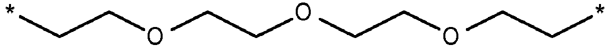 | Formula X6 |

In one embodiment, the composition is characterized in that the hydrophobic radical from formulas X, Xa, Xb, Xb', Xc, Xe, Xf, Xg, Xh and Xi is a radical in which R is a radical from Formula X3.

In one embodiment, the composition is characterized in that the hydrophobic radical from formulas X, Xa, Xb, Xb', Xc, Xe, Xf, Xg, Xh and Xi is a radical in which R is a radical from Formula X4.

In one embodiment, the composition is characterized in that the hydrophobic radical from formulas X, Xa, Xb, Xb', Xc, Xe, Xf, Xg, Xh and Xi is a radical in which R is a polyether radical chosen from the group constituted by the radicals represented by the formulas X5 and X6 below:

In one embodiment, the composition is characterized in that the hydrophobic radical from formulas X, Xa, Xb, Xb', Xc, Xe, Xf, Xg, Xh and Xi is a radical in which R is a polyether radical from Formula X5.

In one embodiment, the composition is characterized in that the hydrophobic radical from formulas X, Xa, Xb, Xb', Xc, Xe, Xf, Xg, Xh and Xi is a radical in which R is a polyether radical from Formula X6.

| | |
|---|---|
| 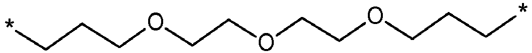 | Formula X5 |
| 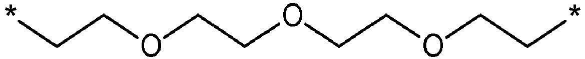 | Formula X6 |

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI' in which G is an alkyl radical comprising 6 carbon atoms represented by formula Z below:

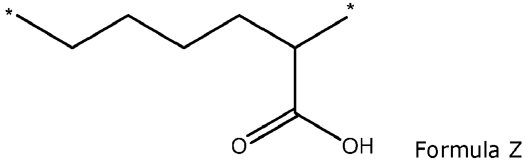

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by formula Z below:

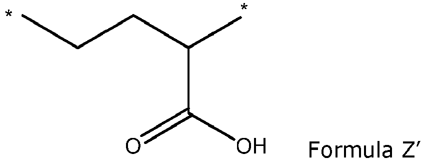

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by -(CH₂)₂-CH(COOH)-.

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by -CH((CH₂)₂COOH)-.

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 3 carbon atoms represented by -CH₂-CH-(COOH).

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 3 carbon atoms represented-CH(CH₂)COOH)-.

In one embodiment, the composition is characterized in that the hydrophobic radical from formulas X, Xa, Xb, Xb', Xc, Xe, Xf, Xg, Xh and Xi is a radical in which GpA is from formula VIII and in which A₁, A₂ or A₃ is chosen from the group constituted by the radicals represented by the formulas below:

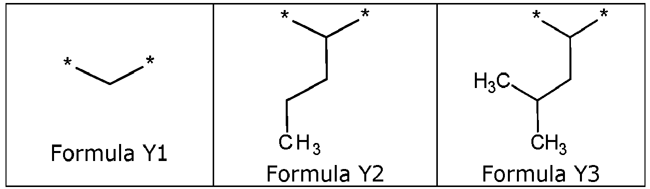

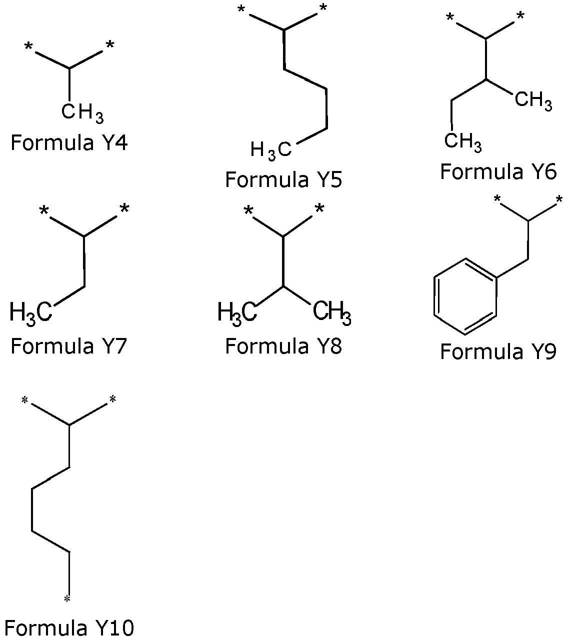

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from the group constituted by the radicals from formulas IXe, IXf or IXg represented below:

| | |
|---|---|
| 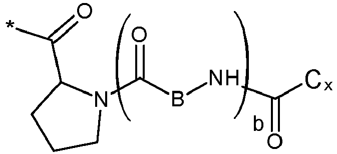 | Formula IXe |
| 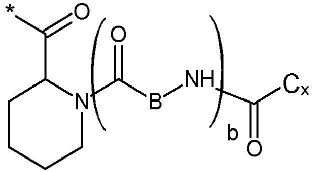 | Formula IXf |
| 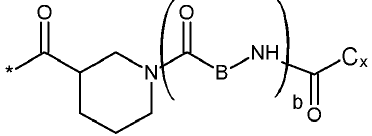 | Formula IXg |

In one embodiment, the composition is characterized in that the hydrophobic radical from formulas X, Xa, Xb, Xb', Xd,Xc, Xd, Xe, Xf, Xg, Xh and Xi is a radical in which the radical GpC from formula IX is chosen from the group constituted by the radicals from formulas IXe, IXf or IXg in which b is equal to 0, respectively responding to formulas IXh, IXi, and IXj below:

| | |
|---|---|
| 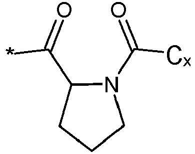 | Formula IXh |
| 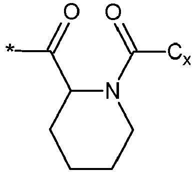 | Formula IXi |
| 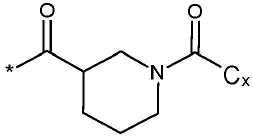 | Formula IXj |

In one embodiment, the composition is characterized in that the hydrophobic radical from formulas X, Xa, Xb, Xb', Xc, Xd, Xe, Xf, Xg, Xh and Xi is a radical in which the radical GpC corresponds to the formula IX or IXe in which b = 0, and corresponds to the formula IXh.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by the linear alkyl radicals.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by branched alkyl radicals.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 19 and 14 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the radicals represented by the formulas below:

| | |
|---|---|
| 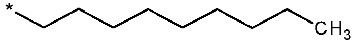 | x = 9 |
| 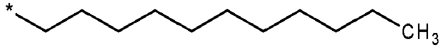 | x = 11 |
| 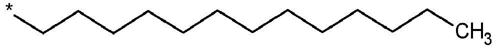 | x = 13 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 15 and 16 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the radicals represented by the formulas below:

| | |
|---|---|
| 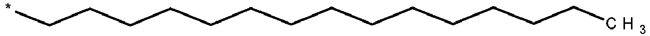 | x = 15 |

In one embodiment, the composition is characterized in that the hydrophobic radical from formulas X, Xa, Xb, Xb', Xc, Xd, Xe, Xf, Xg, Xh and Xi is a radical in which Cx is a radical is chosen from the group constituted by the radicals represented by the formulas below:

| | |
|---|---|
| 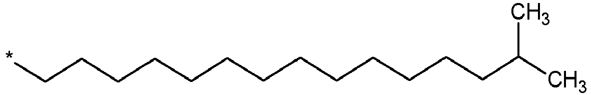 | x = 16 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 17 and 25 carbon atoms.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 17 and 18 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical is chosen from the group constituted by the alkyl radicals represented by the formulas below:

| | |
|---|---|
| 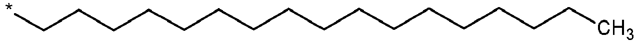 | x = 17 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 18 and 25 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the alkyl radicals represented by the formulas below:

| | |
|---|---|
| 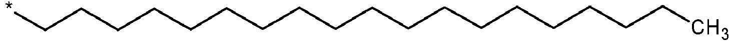 | x = 19 |
| 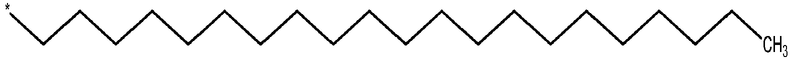 | x = 21 |

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from among the group constituted of radicals in which Cx is chosen from the group constituted by alkyl radicals comprising between 14 and 15 carbon atoms.

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from the group constituted of radicals in which Cx is chosen from the group constituted by the radicals represented by the formulas below:

| | |
|---|---|
| 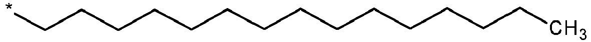 | x = 15 |

In one embodiment, when a' = 1, x is between 11 and 25 (11 ≤ x ≤ 25). In particular, when x is between 15 and 16 (x = 15 or 16) then r = 1 and R is an ether or polyether radical and when x is greater than 17 (x ≥ 17) then r = 1 and R is an ether or polyether radical.

In one embodiment, when a' = 2, x is between 9 and 15 (9 ≤ x ≤ 15).

In one embodiment, the co-polyamino acid is chosen from among the co-polyamino acids of formula XXXb in which the hydrophobic radical -Hy is chosen from the group of hydrophobic radicals from X, Xc', Xa, Xb', Xc, Xe, Xg and Xh in which a' = 1 and I' = 1 and GpC is a radical from formula IXe.

In one embodiment, the co-polyamino acid is chosen from among the co-polyamino acids of formula XXXb in which the hydrophobic radical -Hy is chosen from the group of hydrophobic radicals from formulas X, Xc', Xa, Xb', Xc, Xe, Xg and Xh in which a' = 1 and I' = 1 and GpC is a radical from formula IX in which e=0.

In one embodiment, the co-polyamino acid is chosen from among the co-polyamino acids of formula XXXb in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IXe.

In one embodiment, the co-polyamino acid is chosen from among the co-polyamino acids of formula XXXb in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IX in which e = 0.

In one embodiment, the co-polyamino acid is chosen from among the co-polyamino acids of formula XXXa in which the hydrophobic radical -Hy is chosen from the group of hydrophobic radicals from formulas X, Xc', Xa, Xb', Xc, Xe, Xg and Xh in which a' = 1 and I' = 1 and GpC is a radical from formula IXe.

In one embodiment, the co-polyamino acid is chosen from among the co-polyamino acids of formula XXXa in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IXe.

In one embodiment, the hydrophobic radical Hy is chosen from the group of hydrophobic radicals from formula X, in which h is greater than or equal to 2 h ≥ 2) and GpC is from formula IXe.

In one embodiment, the hydrophobic radical Hy is chosen from the group of hydrophobics from formula X in which g is greater than or equal to 2 (g ≥ 2) and a, I and h are equal to 0 and GpC is from formula IXe.

In one embodiment, the composition is characterized in that the hydrophobic radical from the hydrophobic radicals from formulas X, Xc', Xa, Xb, Xb', Xc, Xe, Xg and Xh in which a' = 1 and I' = 1 and in which Cx is chosen from the group constituted by the linear alkyl radicals.

In one embodiment, the composition is characterized in that the hydrophobic radical is chosen from the hydrophobic radicals from formulas X, Xc', Xa, Xb, Xb', Xc, Xf, Xg and Xi in which a' = 2 or I' = 2 and in which Cx is chosen from the group constituted by the linear alkyl radicals.

In one embodiment, the hydrophobic radical -Hy is chosen from the group of hydrophobic radicals from formula X in which GpR is a radical from formula VII, GpH is a radical from XI and GpC is a radical from formula IX in which e = 1, b = 0 and x = 13.

In a preferred embodiment, hydrophobic radical -Hy is chosen from the group of hydrophobic radicals from formula X 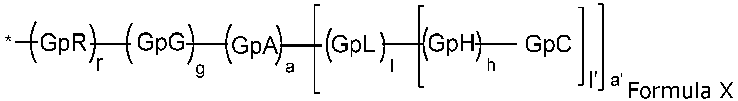 in which:
- r is an integer equal to 0 or to 1, in particular r = 1, and GpR is a radical according to Formula VII 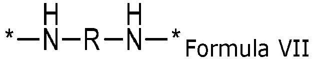
- g = 0,
- a = 0
- a' = 1
- I = 1 and GpL is a radical according to formula XII: 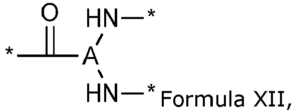
- A is a linear or branched alkyl radicals and/or substituted by a radical derived from a saturated, unsaturated or aromatic ring, comprising 1 to 8 carbon atoms, in particular A is a linear or branched alkyl radical comprising 1 to 8 carbon atoms, more particularly A is a linear alkyl radicals comprising 5 carbon atoms,
- I' = 2
- h = 1, 2 or 3, in particular h = 1, and GpH is a radical according to Formula XI: 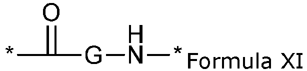
- G is a branched alkyl radical of 1 to 8 carbon atoms, with said alkyl radical bearing one or more free carboxylic acid functions, in particular one free carboxylic acid function,
- GpC is a radical according to formula IX: 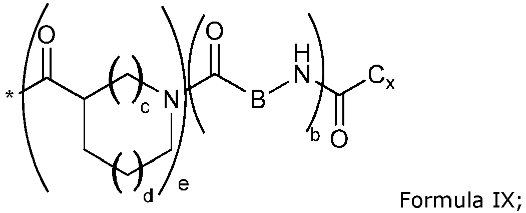 in which b = 0; e = 1; c = 0 or 1; d = 0, 1, or 2; and if c is equal to 0, then d is equal to 1 or to 2; in particular c = 0 and d = 1,
- Cₓ is a linear or branched monovalent alkyl radical, and/or comprising a cyclic part, in which x indicates the number of carbon atoms and when the hydrophobic radical -Hy carries 2 -GpC, then 9 ≤ x ≤ 15, in particular x = 13,
- the hydrophobic radical(s) -Hy according to formula X being bonded to the PLG:
   _{∘} via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
   _{∘} via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0 < M ≤ 0.5;
- when several hydrophobic radicals are borne by a co-polyamino acid, then they are identical or different,
- the degree of polymerization DP in glutamic or aspartic units for PLG chains is comprised from 5 to 250;
- free carboxylic acid functions being in the form of an alkali metal salt chosen from the group consisting of Na⁺ and K⁺.

In one embodiment, the composition according to the invention is characterized in that the M ratio between the number of hydrophobic radicals and the number of glutamate or aspartic unites is between 0.007 and 0.3.

In one embodiment, the composition according to the invention is characterized in that the M ratio between the number of hydrophobic radicals and the number of glutamate or aspartic unites is between 0.01 and 0.3.

In one embodiment, the composition according to the invention is characterized in that the M ratio between the number of hydrophobic radicals and the number of glutamate or aspartic unites is between 0.02 and 0.2.

In one embodiment, the composition according to the invention is characterized in that the hydrophobic radical corresponds to formula X and the M ratio between the number of hydrophobic radicals and the number of glutamate or aspartic unites is between 0.007 and 0.15.

In one embodiment, the composition according to the invention is characterized in that the hydrophobic radical corresponds to formula X and the M ratio between the number of hydrophobic radicals and the number of glutamate or aspartic unites is between 0.01 and 0.1.

In one embodiment, the composition according to the invention is characterized in that the hydrophobic radical corresponds to formula X and the M ratio between the number of hydrophobic radicals and the number of glutamate or aspartic unites is between 0.02 and 0.08.

In one embodiment, the composition according to the invention is characterized in that the hydrophobic radical corresponds to formula X in which radical Cx comprises between 9 and 10 atoms and the M ratio between the number of hydrophobic radicals and the number of glutamate or aspartic unites is between 0.03 and 0.15.

In one embodiment, the composition according to the invention is characterized in that the hydrophobic radical corresponds to formula X in which radical Cx comprises between 11 and 12 atoms and the M ratio between the number of hydrophobic radicals and the number of glutamate or aspartic unites is between 0.015 and 0.1.

In one embodiment, the composition according to the invention is characterized in that the hydrophobic radical corresponds to formula X in which radical Cx comprises between 11 and 12 atoms and the M ratio between the number of hydrophobic radicals and the number of glutamate or aspartic unites is between 0.02 and 0.08.

In one embodiment, the composition according to the invention is characterized in that the hydrophobic radical corresponds to formula X in which radical Cx comprises between 13 and 15 atoms and the M ratio between the number of hydrophobic radicals and the number of glutamate or aspartic unites is between 0.01 and 0.1.

In one embodiment, the composition according to the invention is characterized in that the hydrophobic radical corresponds to formula X in which radical Cx comprises between 13 and 15 atoms and the M ratio between the number of hydrophobic radicals and the number of glutamate or aspartic unites is between 0.01 and 0.06.

In one embodiment, the composition according to the invention is characterized in that the hydrophobic radical corresponds to formula X and the M ratio between the number of hydrophobic radicals and the number of glutamate or aspartic unites is between 0.007 and 0.3.

In one embodiment, the composition according to the invention is characterized in that the hydrophobic radical corresponds to formula X and the M ratio between the number of hydrophobic radicals and the number of glutamate or aspartic unites is between 0.01 and 0.3.

In one embodiment, the composition according to the invention is characterized in that the hydrophobic radical corresponds to formula X and the M ratio between the number of hydrophobic radicals and the number of glutamate or aspartic unites is between 0.015 and 0.2.

In one embodiment, the composition according to the invention is characterized in that the hydrophobic radical corresponds to formula X in which radical Cx comprises between 11 and 14 atoms and the M ratio between the number of hydrophobic radicals and the number of glutamate or aspartic unites is between 0.1 and 0.2.

In one embodiment, the composition according to the invention is characterized in that the hydrophobic radical corresponds to formula X in which radical Cx comprises between 15 and 16 atoms and the M ratio between the number of hydrophobic radicals and the number of glutamate or aspartic unites is between 0.04 and 0.15.

In one embodiment, the composition according to the invention is characterized in that the hydrophobic radical corresponds to formula X in which radical Cx comprises between 17 and 18 atoms and the M ratio between the number of hydrophobic radicals and the number of glutamate or aspartic unites is between 0.02 and 0.06.

In one embodiment, the composition according to the invention is characterized in that the hydrophobic radical corresponds to formula X in which radical Cx comprises between 19 and 25 atoms and the M ratio between the number of hydrophobic radicals and the number of glutamate or aspartic unites is between 0.01 and 0.06.

In one embodiment, the composition according to the invention is characterized in that the hydrophobic radical corresponds to formula X in which radical Cx comprises between 19 and 25 atoms and the M ratio between the number of hydrophobic radicals and the number of glutamate or aspartic unites is between 0.01 and 0.05.

In one embodiment, the composition according to the invention is characterized in that the co-polyamino acid bearing carboxylate charges and hydrophobic radicals is chosen from among the co=poly-amino acids from formula XXXa' below: 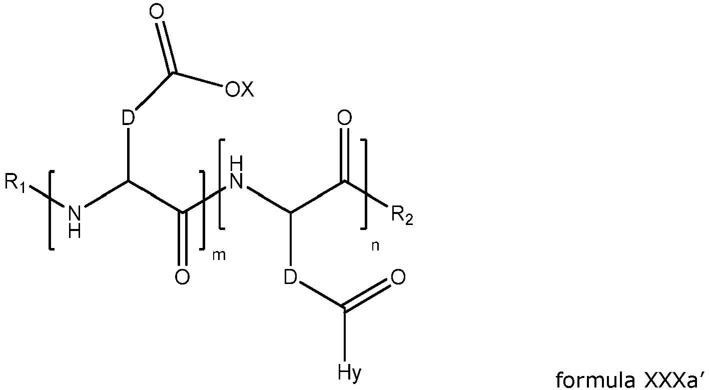 In which
- D represents, independently, either a -CH₂- group (aspartic unit) or a -CH₂-CH₂- group (glutamic unit),
- Hy is a hydrophobic radical chosen from among the hydrophobic radicals from formula X, in which r=1 and GpR is a radical from formula VII,
- R₁ is a hydrophobic radical chosen from among the hydrophobic radicals of formula X in which r=0 or r=1 and GpR is a radical from Formula VII', or a radical chosen from the group constituted by an H, a linear acyl group in C2 to C10, a branched acyl group in C4 to C10, a benzoyl, a terminal "amino acid" and a pyroglutamate,
- R₂ is a hydrophobic radical chosen from among the hydrophobic radicals of formula X in which r=1 and GpR is a radical from Formula VII, or a -NR'R" radical, with R' and R", either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetero-atoms, chosen from the group comprised of O, N and S;
- X represents a cationic entity chosen from the group comprising alkaline cations;
- the sum n + m represents the degree of polymerization DP of the co-polyamino acid, namely, the average number of monomeric units per co-polyamino acid chain and 5 ≤ n + m ≤ 250.

When the co-polyamino acid comprises one or more aspartic units, the latter may be subject to structural re-arrangements.

In one embodiment, the composition according to the invention is characterized in that when the co-polyamino acid comprises aspartic units, then the co-polyamino acid may also comprise monomeric units from formula XXXI and/or XXXI'.

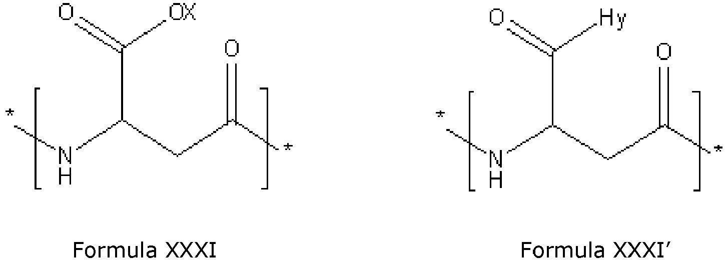

We call "a statistically grafted co-polyamino acid" a co-polyamino acid bearing carboxylate charges and at least one hydrophobic radical, a co-polyamino acid of formula XXXa.

In one embodiment, the composition according to the invention is characterized in that the co-polyamino acid bearing carboxylate charges and hydrophobic radicals is chosen from among the co-polyamino acids from formula XXXa', in which R₁ = R'₁ and R₂ = R'₂, from formula XXXa below: 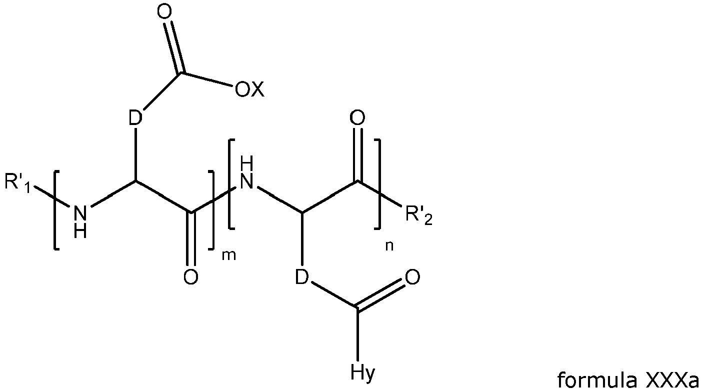 in which
- m, n, X, D and Hy have the definitions given above,
- R'₁ is a radical chosen from among the group constituted by an H, a linear acyl group in C2 to C10, a branched acyl group in C4 to C10, a benzoyl, a terminal "amino acid" and a pyroglutamate,
- R'₂ is a -NR'R" radical with R' and R" , either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetero-atoms, chosen from the group comprised of O, N and S.

In one embodiment, the composition according to the invention is characterized in that the co-polyamino acid bearing carboxylate charges and hydrophobic radicals is chosen from among the co-polyamino acids from formula XXXa in which Hy is a radical from formula X.

In one embodiment, the composition according to the invention is characterized in that the co-polyamino acid bearing carboxylate charges and hydrophobic radicals is chosen from among the co-polyamino acids from formula XXXa in which Hy is a radical from formula X, in which r=1.

In one embodiment, the composition according to the invention is characterized in that the co-polyamino acid bearing carboxylate charges and hydrophobic radicals is chosen from among the co-polyamino acids from formula XXXa, in which Hy is a radical from formula X, in which r=1, and for GpC, b=0.

In one embodiment, the composition according to the invention is characterized in that the co-polyamino acid bearing carboxylate charges and hydrophobic radicals is chosen from among the co-polyamino acids from formula XXXa in which Hy is a radical from formula X and in which GpC is a radical from formula IX.

In one embodiment, the composition according to the invention is characterized in that the co-polyamino acid bearing carboxylate charges and hydrophobic radicals is chosen from among the co-polyamino acids from formula XXXa in which Hy is a radical from formula X and in which GpC is a radical from formula IX and r=1.

We call "a semi-telechelic grafted co-polyamino acid" a co-polyamino acid bearing carboxylate charges and at least one hydrophobic radical, a co-polyamino acid of formula XXXb.

In one embodiment, the composition according to the invention is characterized in that the co-polyamino acid bearing carboxylate charges and hydrophobic radicals is chosen from among the co-polyamino acids from formula XXXa', in which n=0 from the following formula XXXb: 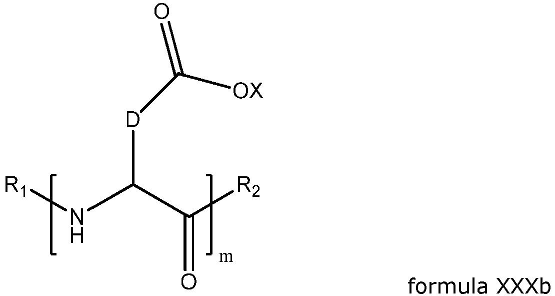 In which m, X, D, R₁ and R₂ have the definitions given above and at least R₁ or R₂ is a hydrophobic radical from formula X.

In one embodiment the composition according to the invention is characterized in that the co-polyamino acid bearing carboxylate charges and hydrophobic radicals is chosen from among the co-polyamino acids from formula XXXa' in which n=0 of formula XXXb and R₁ or R₂ is a hydrophobic radical from formula X.

In one embodiment, the composition according to the invention is characterized in that the co-polyamino acid bearing carboxylate charges and hydrophobic radicals is chosen from among the co-polyamino acids from formula XXXb in which R1= R'1 from formula XXXb': 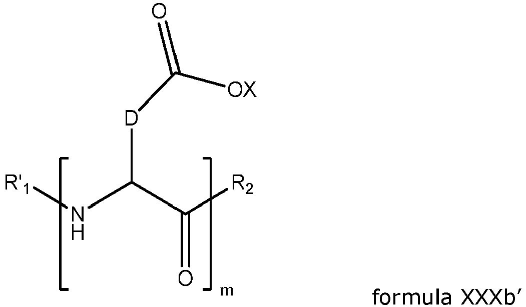 in which m, X, D, R'₁ and R₂ have the definitions given above and R₂ is a hydrophobic radical from formula X.

In one embodiment, the composition according to the invention is characterized in that the co-polyamino acid bearing carboxylate charges and hydrophobic radicals is chosen from among the co-polyamino acids from formula XXXb in which R₂ = R'₂ from formula XXXb": 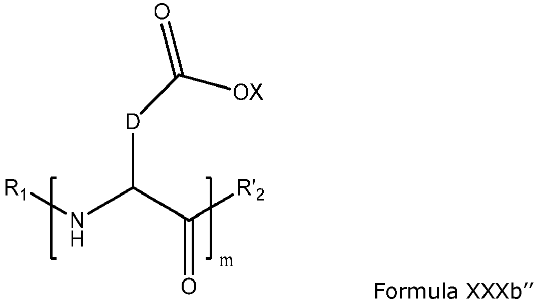 in which m, X, D, R₁ and R'₂ have the definitions given above and R₁ is a hydrophobic radical from formula X.

In one embodiment, the composition according to the invention is characterized in that the co-polyamino acid bearing carboxylate charges and hydrophobic radicals is chosen from among the co-polyamino acids from formula XXXb or XXXb" in which R₁ is a radical from formula X and in which GpR is from formula VII'.

In one embodiment, the composition according to the invention is characterized in that the co-polyamino acid bearing carboxylate charges and hydrophobic radicals is chosen from among the co-polyamino acids from formula XXXb or XXXb" in which R₁ is a radical from formula X and in which GpR is a from formula VII".

In one embodiment, the composition according to the invention is characterized in that the co-polyamino acid bearing carboxylate charges and hydrophobic radicals is chosen from among the co-polyamino acids from formula XXXb or XXXb" in which R₁ is a hydrophobic radical from formula X and in which GpR is from formula VII' and GpC is from formula IX.

In one embodiment, the composition according to the invention is characterized in that the co-polyamino acid bearing carboxylate charges and hydrophobic radicals is chosen from among the co-polyamino acids from formula XXXb or XXXb" in which R₁ is a hydrophobic radical from formula X and in which GpR is from formula VII' and GpC is from formula IX.

In one embodiment, the composition according to the invention is characterized in that the co-polyamino acid bearing carboxylate charges and hydrophobic radicals is chosen from among the co-polyamino acids from formula XXXb or XXXb' in which R₂ is a hydrophobic radical from formula X and in which r = 1 and GpR is from formula VII.

In one embodiment, the composition according to the invention is characterized in that the co-polyamino acid bearing carboxylate charges and hydrophobic radicals is chosen from among the co-polyamino acids from formula XXXa' in which at least one of R₁ or R₂ is a hydrophobic radical as defined above from the following formula XXX: 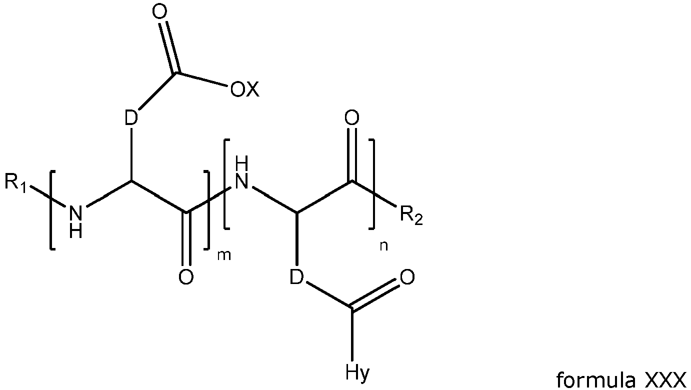 In which
- D represents, independently, either a -CH₂- group (aspartic unit) or a -CH₂-CH₂-group (glutamic unit),
- Hy is a hydrophobic radical chosen from among the hydrophobic radicals from formula X, in which r=1 and GpR is a radical from formula VII,
- R₁ is a hydrophobic radical chosen from among the hydrophobic radicals of formula X in which r=0 or r=1 and GpR is a radical from Formula VII', or a radical chosen from the group constituted by an H, a linear acyl group in C2 to C10, a branched acyl group in C4 to C10, a benzoyl, a terminal "amino acid" unit and a pyroglutamate,
- R₂ is a hydrophobic radical chosen from among the hydrophobic radicals of formula X in which r=1 and GpR is a radical from Formula VII, or a -NR'R" radical, with R' and R", either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetro-atoms, chosen from the group comprised of O, N and S,
- X represents an H or a cationic entity chosen from the group comprising the metallic cations;
- the sum n + m represents the degree of polymerization DP of the co-polyamino acid, namely, the average number of monomeric units per co-polyamino acid chain and 5 ≤ n + m ≤ 250.

In one embodiment', the composition according to the invention is characterized in that the co-polyamino acid bearing carboxylate charges and hydrophobic radicals is chosen from among the co-polyamino acids from formula XXX, XXXa, XXXa', XXXb, XXXb' or XXXb" in which group D is a -CH₂- group (aspartic unit).

In one embodiment, the composition according to the invention is characterized in that the co-polyamino acid bearing carboxylate charges and hydrophobic radicals is chosen from among the co-polyamino acids from formula XXX, XXXa, XXXa', XXXb, XXXb' or XXXb" in which group D is a -CH₂-CH₂- group (glutamic unit).

In one embodiment, the composition according to the invention is characterized in that R₁ is a radical chosen from the group comprised by a linear acyl group in C₂ to C₁₀, a branched acyl group in C₄ to C₁₀, a benzoyl, a terminal "amino acid" unit and a pyroglutamate.

In one embodiment, the composition according to the invention is characterized in that R₁ is a radical chosen from the group comprised by a linear acyl group in C₂ to C₁₀, or a branched acyl group in C₄ to C₁₀.

In one embodiment, the composition according to the invention is characterized in that the co-polyamino acid bearing carboxylate charges and hydrophobic radicals is chosen from among the co-polyamino acids from formula XXXa, XXXb, XXXb' or XXXb"in which group D is a -CH₂- group (aspartic unit).

In one embodiment, the composition according to the invention is characterized in that the co-polyamino acid bearing carboxylate charges and hydrophobic radicals is chosen from among the co-polyamino acids from formulas XXXa, XXXb, XXXb' or XXXb" in which the co-polyamino acid is chosen from among the co-polyamino acids in which group D is a -CH₂-CH₂- group (glutamic unit).

In one embodiment, the composition according to the invention is characterized in that n+m is between 10 and 200.

In one embodiment, the composition according to the invention is characterized in that n+m is between 15 and 150.

In one embodiment, the composition according to the invention is characterized in that n+m is between 15 and 100.

In one embodiment, the composition according to the invention is characterized in that n+m is between 15 and 80.

In one embodiment, the composition according to the invention is characterized in that n+m is between 15 and 65.

In one embodiment, the composition according to the invention is characterized in that n+m is between 20 and 60.

In one embodiment, the composition according to the invention is characterized in that n+m is between 20 and 50.

In one embodiment, the composition according to the invention is characterized in that n+m is between 20 and 40.

In a preferred embodiment, the composition comprises a co-polyamino acid according to formula XXXb' or XXXb", in particular XXXb': 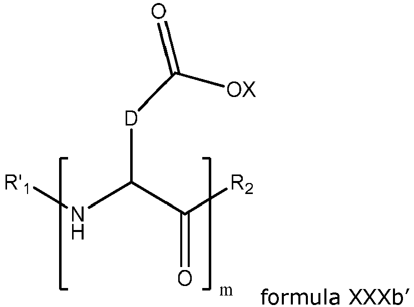 in which m, X, D, R'₁ and R₂ have the definitions given above and R₂ is a hydrophobic radical -Hy, or 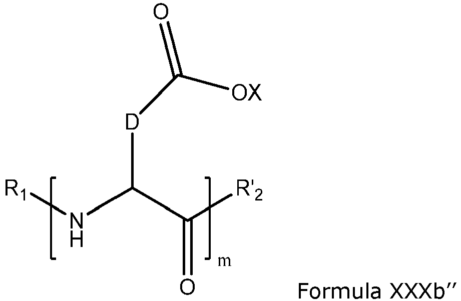 in which m, X, D, R₁ and R'₂ have the definitions given above and R₁ is a hydrophobic radical -Hy,
said radical -Hy is chosen from the group of hydrophobic radicals from formula X 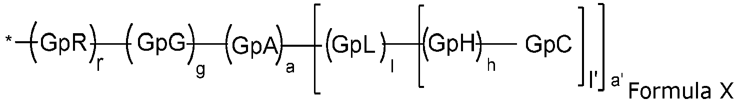 in which:
- r is an integer equal to 0 or to 1, in particular r = 1, and GpR is a radical according to Formula VII 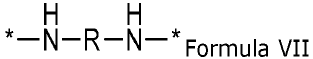
- g = 0,
- a = 0
- a' = 1
- I = 1 and GpL is a radical according to formula XII: 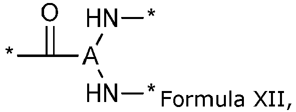
- A is a linear or branched alkyl radical and/or substituted by a radical derived from a saturated, unsaturated or aromatic ring, comprising 1 to 8 carbon atoms, in particular A is a linear or branched alkyl radical comprising 1 to 8 carbon atoms, more particularly A is a linear alkyl radical comprising 5 carbon atoms,
- I' = 2
- h = 1, 2 or 3, in particular h = 1, and GpH is a radical according to Formula XI: 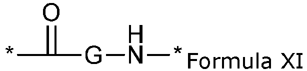
- G is a branched alkyl radical of 1 to 8 carbon atoms, with said alkyl radical bearing one or more free carboxylic acid functions, in particular one free carboxylic acid function,
- GpC is a radical according to formula IX: 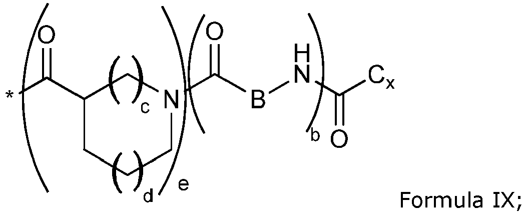 in which b = 0; e = 1; c = 0 or 1; d = 0, 1, or 2; and if c is equal to 0, then d is equal to 1 or to 2; in particular c = 0 and d = 1,
- Cₓ is a linear or branched monovalent alkyl radical, and/or comprising a cyclic part, in which x indicates the number of carbon atoms and when the hydrophobic radical -Hy carries 2 -GpC, then 9 ≤ x ≤ 15, in particular x = 13,
- the hydrophobic radical(s) -Hy according to formula X being bonded to the PLG:
   _{∘} via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
   _{∘} via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0.01 < M ≤ 0.4, in particular from 0.02 to 0.1,
- when several hydrophobic radicals are borne by a co-polyamino acid, then they are identical or different,
- the degree of polymerization DP in glutamic or aspartic units for PLG chains is comprised from 5 to 250, in particular from 10 to 50,
- free carboxylic acid functions being in the form of an alkali metal salt chosen from the group consisting of Na⁺ and K⁺.

In a most preferred embodiment, the composition comprises a co-polyamino acid as represented below: 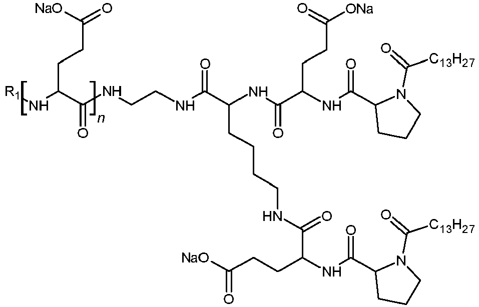 M = 0.02 to 0.1, DP or n = 10 to 50, R₁ = H or pyroglutamate

In a preferred embodiment the composition comprises a co-polyamino acid as defined in the 2 paragraphs above.

According to an embodiment, the co-polyamino acid is as defined in WO2019110774.

In one embodiment, the composition according to the invention is characterized in that the co-polyamino acid is the result of a polyamino acid obtained by polymerization.

In one embodiment, the composition according to the invention is characterized in that the co-polyamino acid is the result of a polyamino acid obtained by ring-opening polymerization of a N-carboxy anhydride derivative of glutamic acid or of a N-carboxy anhydride derivative of aspartic acid.

In one embodiment, the composition according to the invention is characterized in that the co-polyamino acid is the result of a polyamino acid obtained by ring-opening polymerization of a *N*-carboxyanhydride derivative of glutamic acid or of a *N*-carboxy anhydride derivative of aspartic acid as described in the article from the journal Adv. Polym. Sci. 2006, 202, 1-18 (Deming, T.J.).

In one embodiment, the composition according to the invention is characterized in that the co-polyamino acid is the result of a polyamino acid obtained by ring opening polymerization of a N-carboxy anhydride derivative of glutamic acid.

In one embodiment, the composition according to the invention is characterized in that the co-polyamino acid comes from a polyamino acid obtained by ring-opening polymerization of a N-carboxy anhydride derivative of glutamic acid chosen from the group constituted by *N*-carboxyanhydride methyl glutamate (GluOMe-NCA), N-carboxy anhydride benzyl glutamate (GluOBzl-NCA) and N-carboxy anhydride t-butyl glutamate (GluOtBu-NCA).

In one embodiment, the N-carboxy anhydride glutamic acid derivative is methyl L-glutamate N-carboxy anydride (L-GluOMe-NCA).

In one embodiment, the N-carboxy anhydride glutamic acid derivative is benzyl L-glutamate N-carboxy anydride (L-GluOBzl-NCA).

In one embodiment, the composition according to the invention is characterized in that the co-polyamino acid comes from a polyamino acid obtained by ring opening polymerization of a *N*-carboxyanhydride derivative of glutamic acid or a *N*-carboxyanhydride derivative of aspartic acid using an organo-metallic compound of a transition metal as an initiator as described in the publication Nature 1997, 390, 386-389 (Deming, T. J.).

In one embodiment, the composition according to the invention is characterized in that the co-polyamino acid is the result of a polyamino acid obtained by ring opening polymerization of a *N*-carboxyanhydride derivative of glutamic acid or a *N*-carboxyanhydride derivative of aspartic acid using ammonia or a primary amine as described in French patent FR 2,801,226 (Touraud, F., et al.) and the references cited by this patent.

In one embodiment, the composition according to the invention is characterized in that the co-polyamino acid is the result of a polyamino acid obtained by ring opening polymerization of a *N*-carboxyanhydride derivative of glutamic acid or a *N*-carboxyanhydride derivative of aspartic acid using hexamethyldisilazide as described in the publication J. Am. Chem. Soc. 2007, 129, 14114-14115 (Lu H. ; et al.) or a silylated amine as described in the publication J. Am. Chem. Soc. 2008, 130, 12562-12563 (Lu H. ; et al.).

In one embodiment, the composition according to the invention is characterized in that the process for synthesizing the polyamino acid obtained by ring opening polymerization of a *N*-carboxyanhydride derivative of glutamic acid or a *N-*carboxyanhydride derivative of aspartic acid from which the co polyamino acid results comprises a stage of ester functions hydrolysis.

In one embodiment, this step of ester functions hydrolysis may consist of hydrolysis in an acidic medium or hydrolysis in a basic medium or be carried out by hydrogenolysis.

In one embodiment, this ester groups hydrolysis step is carried out by hydrolysis in an acidic medium.

In one embodiment, this ester groups hydrolysis step is carried out by hydrolysis in a basic medium.

In one embodiment, this ester groups hydrolysis step is carried out by hydrogenolysis.

In one embodiment, the composition according to the invention is characterized in that the co-polyamino acid is the result of a polyamino acid obtained by depolymerization of a polyamino acid of a higher molecular weight.

In one embodiment, the composition according to the invention is characterized in that the co-polyamino acid is the result of a polyamino acid obtained by enzymatic depolymerization of a polyamino acid of a higher molecular weight.

In one embodiment, the composition according to the invention is characterized in that the co-polyamino acid is the result of a polyamino acid obtained by chemical depolymerization of a polyamino acid of a higher molecular weight.

In one embodiment, the composition according to the invention is characterized in that the co-polyamino acid is the result of a polyamino acid obtained by enzymatic and chemical depolymerization of a polyamino acid of a higher molecular weight.

In one embodiment, the composition according to the invention is characterized in that the co-polyamino acid is the result of a polyamino acid obtained by depolymerization of a polyamino acid of a higher molecular weight chosen from the group constituted by sodium polyglutamate and sodium polyaspartate.

In one embodiment, the composition according to the invention is characterized in that the co-polyamino acid is the result of a polyamino acid obtained by depolymerization of a sodium polyglutamate of a higher molecular weight.

In one embodiment, the composition according to the invention is characterized in that the co-polyamino acid is the result of a polyamino acid obtained by depolymerization of a sodium polyaspartate of a higher molecular weight.

In one embodiment, the composition according to the invention is characterized in that the co-polyamino acid is obtained by grafting a hydrophobic group onto a poly-L-glutamate acid or a poly-L-aspartate acid by using the amide bond formation process well-known to the person versed in the art.

In one embodiment, the composition according to the invention is characterized in that the co-polyamino acid is obtained by grafting a hydrophobic group onto a poly-L-glutamate acid or a poly-L-aspartate acid by using the amide bond formation process used for peptide synthesis.

In one embodiment, the composition according to the invention is characterized in that the co-polyamino acid is obtained by grafting a hydrophobic group onto a poly-L-glutamate acid or a poly-L-aspartate acid as described in French patent FR 2,840,614 (Chan, Y.P.; et al.).

In one embodiment, the concentration of co-polyamino acid bearing carboxylate charges and hydrophobic radicals is at most 60 mg/ml.

In one embodiment, the concentration of co-polyamino acid bearing carboxylate charges and hydrophobic radicals is at most 40 mg/ml.

In one embodiment, the concentration of co-polyamino acid bearing carboxylate charges and hydrophobic radicals is at most 20 mg/ml.

In one embodiment, the concentration of co-polyamino acid bearing carboxylate charges and hydrophobic radicals is at most 10 mg/ml.

In one embodiment, the concentration of co-polyamino acid bearing carboxylate charges and hydrophobic radicals is at most 5 mg/ml.

In one embodiment, the concentration of co-polyamino acid bearing carboxylate charges and hydrophobic radicals is at most 2.5 mg/ml.

In one embodiment, the concentration of co-polyamino acid bearing carboxylate charges and hydrophobic radicals is ranging from 2.5 to 60 mg/ml.

In one embodiment, the concentration of co-polyamino acid bearing carboxylate charges and hydrophobic radicals is ranging from 5 to 50 mg/ml.

In one embodiment, the concentration of co-polyamino acid bearing carboxylate charges and hydrophobic radicals is ranging from 7.5 to 40 mg/ml.

In one embodiment, the concentration of co-polyamino acid bearing carboxylate charges and hydrophobic radicals is ranging from 10 to 30 mg/ml.

According to an embodiment, the composition does not comprise a basal insulin for which the isoelectric point, pI, is comprised from 5.8 to 8.5.

According to an embodiment, the composition does not comprise insulin glargine.

According to an embodiment, the composition does not comprise human amylin.

According to an embodiment, the composition does not comprise pramlintide.

According to an embodiment, the composition does not comprise glucagon.

According to an embodiment, the composition does not comprise human glucagon.

Semaglutide is a GLP-1 receptor agonist also known as N<626>-{18-[N-(17-carboxyheptadecanoyl)-L-y-glutamyl]-10-oxo-3,6,12,15-tetraoxa-9,18-diazaoctadecanoyl}-[8-(2-amino-2-propanoic acid),34-L-arginine]human glucagon-like peptide 1 (7-37). Semaglutide was described in WO2006/097537 and in J. Med. Chem. 2015, 58, 18, 7370-7380 and may be manufactured using methods well known to the person skilled in the art, such as that briefly described in WO2006/097537, Example 4.

Semaglutide may be present in the composition in its fully or partly ionized form; for example, one or more carboxylic acid groups (-COOH) may be deprotonated into the carboxylate group -COO⁻ and/or one or more amino groups (-NH₂) may be protonated into the -NH₃⁺ group.

Semaglutide may be in the form of a salt, preferably a pharmaceutically acceptable salt.

When the potency of semaglutide was tested according to the procedure described in WO/2022/129526, Assay 1, semaglutide had an EC50 value of about 5.5 pM (see WO/2022/129526, Tables 4b and 4c). The concentration of semaglutide in the pharmaceutical formulation disclosed herein may be from about 0.25 mg/ml to about 22 mg/ml.

The concentration of semaglutide in the compositions disclosed herein may be from about 0.25 mg/ml to about 22 mg/ml.

In an embodiment, the semaglutide is comprised in a concentration ranging from 0.3 to 20 mg/ml.

In an embodiment, the semaglutide is comprised in a concentration ranging from 0.25 to 0.5 mg/ml, in particular is about 0.37 mg/ml.

In an embodiment, the semaglutide is comprised in a concentration ranging from 0.5 to 1.0 mg/ml, in particular is about 0.75 mg/ml.

In an embodiment, the semaglutide is comprised in a concentration ranging from 1.0 to 1.5 mg/ml, in particular is about 1.25 mg/ml.

In an embodiment, the semaglutide is comprised in a concentration ranging from 1.5 to 2.0 mg/ml.

In an embodiment, the semaglutide is comprised in a concentration ranging from 2.0 to 2.5 mg/ml.

In an embodiment, the semaglutide is comprised in a concentration ranging from 2.5 to 3.0 mg/ml.

In an embodiment, the semaglutide is comprised in a concentration ranging from 3.0 to 3.5 mg/ml, in particular is about 3.2 mg/ml.

In an embodiment, the semaglutide is comprised in a concentration ranging from 3.5 to 4.0 mg/ml.

In an embodiment, the semaglutide is comprised in a concentration ranging from 4.0 to 5.0 mg/ml.

In an embodiment, the semaglutide is comprised in a concentration ranging from 5.0 to 7.5 mg/ml.

In an embodiment, the semaglutide is comprised in a concentration ranging from 7.5 to 10 mg/ml.

In an embodiment, the semaglutide is comprised in a concentration ranging from 10 to 15 mg/ml.

In an embodiment, the semaglutide is comprised in a concentration ranging from 15 to 20 mg/ml.

In an embodiment, the semaglutide is comprised in a concentration ranging from 20 to 25 mg/ml.

Semaglutide has a theoretically calculated isoelectric point of about 4.37.

In a preferred embodiment, the cagrilintide define cagrilintide or a biologically active metabolite or degradation product of cagrilintide.

In a most preferred embodiment, cagrilintide means cagrilintide alone, i.e not comprising its biologically active metabolites or degradation products.

Cagrilintide has a theoretically calculated isoelectric point of about 8.56.

Cagrilintide, also called AM833, is disclosed as Example 53 in WO2012/168432: N-alpha-[(S)-4-Carboxy-4-(19-carboxynonadecanoylamino)butyryl]-[Glu14,Arg17,Pro37]-pramlintide. Cagrilintide may be prepared as described in WO2012/168432, pages 153-155.

Cagrilintide may be in the form of a salt, preferably a pharmaceutically acceptable salt.

A biologically active metabolite or degradation product of cagrilintide may have an aspartate (Asp) in position 21 or 22. A biologically active metabolite or degradation product of cagrilintide may have an iso-aspartate (iso-Asp) in position 21 or 22.

When the potency of cagrilintide was tested using the procedure described in WO2022129526, Assay 2, cagrilintide had an EC50value of about 11 pM (WO2022/129526, Tables 4b and 4c).

In an embodiment, the concentration of cagrilintide is going from about 0.25 mg/ml to about 22 mg/ml.

In an embodiment, the concentration of cagrilintide is going from 0.3 to 20 mg/ml.

In an embodiment, the concentration of cagrilintide is going from about 0.25 to 0.5 mg/ml, in particular is about 0.37 mg/ml.

In an embodiment, the concentration of cagrilintide is going from about 0.5 to 1.0 mg/ml, in particular is about 0.75 mg/ml.

In an embodiment, the concentration of cagrilintide is going from about 1.0 to 1.5 mg/ml, in particular is about 1.25 mg/ml.

In an embodiment, the cagrilintide is comprised in a concentration ranging from 1.5 to 2.0 mg/ml.

In an embodiment, the cagrilintide is comprised in a concentration ranging from 2.0 to 2.5 mg/ml.

In an embodiment, the cagrilintide is comprised in a concentration ranging from 2.5 to 3.0 mg/ml.

In an embodiment, the cagrilintide is comprised in a concentration ranging from 3.0 to 3.5 mg/ml, in particular about 3.25 mg/ml.

In an embodiment, the cagrilintide is comprised in a concentration ranging from 3.5 to 4.0 mg/ml.

In an embodiment, the cagrilintide is comprised in a concentration ranging from 4.0 to 5.0 mg/ml.

In an embodiment, the cagrilintide is comprised in a concentration ranging from 5.0 to 7.5 mg/ml.

In an embodiment, the cagrilintide is comprised in a concentration ranging from 7.5 to 10 mg/ml.

In an embodiment, the cagrilintide is comprised in a concentration ranging from 10 to 15 mg/ml.

In an embodiment, the cagrilintide is comprised in a concentration ranging from 15 to 20 mg/ml.

In an embodiment, the cagrilintide comprised in a concentration ranging from 20 to 25 mg/ml.

In an embodiment, the weight ratio semaglutide / cagrilintide- is going from 0.1 to 10.

In an embodiment, the weight ratio semaglutide / cagrilintide is going from 0.2 to 5.

In an embodiment, the weight ratio semaglutide / cagrilintide is going from 0.3 to 3.

In an embodiment, the weight ratio semaglutide / cagrilintide is going from 0.5 to 2.

In an embodiment, the weight ratio semaglutide / cagrilintide is going from 0.8 to 1.2.

In an embodiment, the weight ratio semaglutide / cagrilintide is going from 0.9 to 1.1.

In an embodiment, the weight ratio semaglutide / cagrilintide is about 1.

In an embodiment the compositions comprise 3.2 mg of cagrilintide and 3.2 mg of semaglutide.

The pH of compositions according to the invention is comprised in a range from 5.0 to 6.0.

According to an embodiment the pH of 6.0 can be disclaimed from the ranges of pH.

In an embodiment the pH is comprised in a range from 5.0 to 5.9.

In an embodiment the pH is comprised in a range from 5.1 to 5.9.

In an embodiment the pH is comprised in a range from 5.2 to 5.9.

In an embodiment the pH is comprised in a range from 5.2 to 5.8.

In an embodiment the pH is comprised in a range from 5.2 and 5.7.

In an embodiment the pH is comprised in a range from 5.2 and 5.6.

In an embodiment the pH is comprised in a range from 5.3 and 5.5.

In an embodiment the pH is about 5.4.

In one embodiment, the hydrophobic radical to semaglutide and cagrilintide molar ratio [Hy]/ [semaglutide] + [cagrilintide] ≤ 6.

In one embodiment, the hydrophobic radical to semaglutide and cagrilintide molar ratio [Hy]/ [semaglutide] + [cagrilintide] ≤ 5.

In one embodiment, the hydrophobic radical to semaglutide and cagrilintide molar ratio [Hy]/ [semaglutide] + [cagrilintide] ≤ 4.

In one embodiment, the hydrophobic radical to semaglutide and cagrilintide molar ratio [Hy]/ [semaglutide] + [cagrilintide] ≤ 3.

In one embodiment, the hydrophobic radical to semaglutide and cagrilintide molar ratio [Hy]/ [semaglutide] + [cagrilintide] ≥ 1.

In one embodiment, the hydrophobic radical to semaglutide and cagrilintide molar ratio [Hy]/ [semaglutide] + [cagrilintide] ≥ 1.5.

In one embodiment, the hydrophobic radical to semaglutide and cagrilintide molar ratio [semaglutide] + [cagrilintide] ≥ 2.

In one embodiment, the hydrophobic radical to semaglutide and cagrilintide molar ratio [Hy]/ [semaglutide] + [cagrilintide] ≥ 2.5.

In one embodiment, the compositions according to the invention also comprise buffers.

In one embodiment, the composition according to the invention comprises buffers at concentrations between 0 and 100 mM.

In one embodiment, the composition according to the invention comprises buffers at concentrations between 15 and 50 mM.

In one embodiment, compositions according to the invention comprise a buffer chosen from the group constituted by a phosphate buffer, Tris (trishydroxymethyl-aminomethane).

In one embodiment, the buffer is sodium phosphate.

In one embodiment, the buffer is Tris (trishydroxymethyl-aminomethane).

In one embodiment, the buffer is sodium citrate.

In one embodiment, the compositions according to the invention also comprise a preservative.

In one embodiment, the preservatives are chosen from the group constituted by m-cresol and phenol, alone or in mixture.

In one embodiment, the concentration of the preservatives is between 10 and 50 mM.

In one embodiment, the concentration of the preservatives is between 10 and 40 mM.

In one embodiment, the compositions according to the invention also comprise a surfactant.

In one embodiment, the surfactant is chosen from the group constituted by propylene glycol and polysorbate.

Compositions according to the invention may also comprise additives such as osmotic agents.

In one embodiment, the osmotic agents are chosen from the group constituted by glycerin, sodium chloride, mannitol and glycine.

Compositions according to the invention may also comprise any pharmacopoeia compatible excipients which are also compatible with cagrilintide and semaglutide, at the usage concentrations.

The compositions or formulations disclosed herein are suitable for parenteral injection, in particular for subcutaneous injection.

In the case of local and systemic releases, the administration pathways foreseen are intravenous, subcutaneous, intra-dermal or intramuscular.

In a preferred embodiment the administration is subcutaneous.

In one embodiment, the composition according to the invention is characterized in that it is administered twice a week.

In one embodiment, the composition according to the invention is characterized in that it is administered once a week.

In one embodiment, the composition according to the invention is characterized in that it is administered twice a month.

The invention also relates to single-dose formulations at a pH between 5.2 and 6.0 and comprising cagrilintide, semaglutide and co-polyamino acid as defined above.

In one embodiment, the formulations are in the form of an injectable solution.

If necessary, the composition of the mixture of cagrilintide, semaglutide and the co-polyamino acid is adjusted with excipients such as glycerin, m-cresol, and polysorbate (Tween^{®}) by addition of concentrated solutions of these excipients to the mixture. If necessary, the pH of the preparation is adjusted to a pH between 5.0 and 6.0.

According to an embodiment, the composition is in the form of an aqueous injectable solution, whose pH is comprised from 5.0 to 6.0, in particular 5.1 to 5.9, comprising:
- semaglutide,
- cagrilintide, and
- a co-polyamino acid (PLG) bearing carboxylate charges and hydrophobic radicals Hy having the following Formula 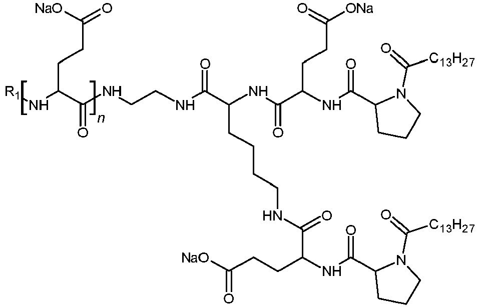 M = 0.02 to 0.1, DP = 10 to 50, R₁ = H or pyroglutamate.

According to an embodiment, the composition is in the form of an aqueous injectable solution, whose pH is comprised from 5.2 to 5.8, comprising:
- semaglutide,
- cagrilintide, and
- a co-polyamino acid (PLG) bearing carboxylate charges and hydrophobic radicals Hy having the following Formula : 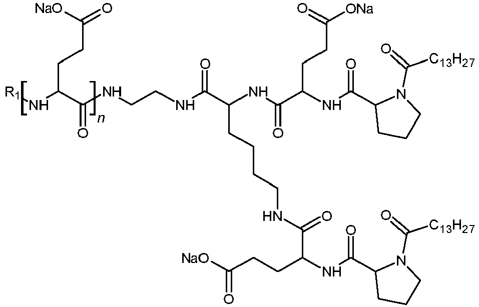 M = 0.02 to 0.1, DP or n = 10 to 50, R₁ = H or pyroglutamate.

The instant invention further concerns compositions for use as a medicament.

In an embodiment, the invention concerns a composition, in the form of an aqueous injectable solution, for use as a medicament, whose pH is comprised from 5.0 to 6.0, in particular from 5.1 to 5.9, comprising:
- semaglutide,
- cagrilintide, and
- a co-polyamino acid (PLG) bearing carboxylate charges and hydrophobic radicals Hy having the following Formula: 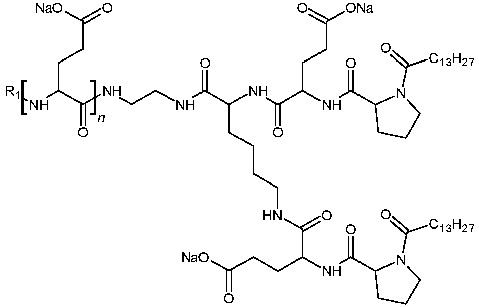 M = 0.02 to 0.1, n = 10 to 50, R₁ = H or pyroglutamate.

In an embodiment, the invention concerns a composition, in the form of an aqueous injectable solution, for use as a medicament, whose pH is comprised from 5.2 to 5.8, comprising:
- semaglutide,
- cagrilintide, and
- a co-polyamino acid (PLG) bearing carboxylate charges and hydrophobic radicals Hy having the following Formula: 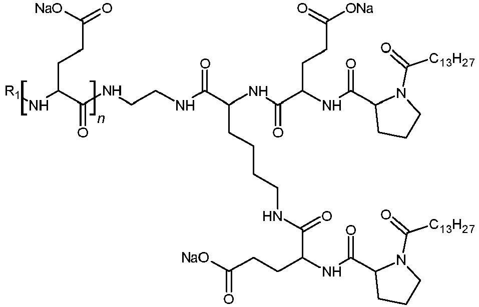 M = 0.02 to 0.1, n = 10 to 50, R₁ = H or pyroglutamate.

The invention also concerns compositions for use in a method of treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

In an embodiment, compositions are for use in a method of treatment of overweight or obesity.

In an embodiment, compositions are for use in a method of treatment of diabetes.

In an embodiment, compositions are for use in a method of treatment of Diabetes Type 1.

In an embodiment, compositions are for use in a method of treatment of Diabetes Type 2.

In an embodiment, compositions are for use in a method of treatment of overweight or obesity and diabetes.

In an embodiment, compositions are for use in a method of treatment of overweight or obesity and Diabetes Type 1.

In an embodiment, compositions are for use in a method of treatment of overweight or obesity and Diabetes Type 2.

In an embodiment, compositions are for use in a method of treatment of cardio-vascular disease(s).

In an embodiment, compositions are for use in a method of treatment of non-alcoholic steatohepatitis (NASH).

The invention further concerns a method for the preparation of a stable, injectable aqueous solutions of semaglutide comprising the step of mixing a co-polyamino acid as previously defined with semaglutide.

In an embodiment, the method is for the preparation of a stable, injectable aqueous solutions of semaglutidecomprising the step of mixing a co-polyamino acid (PLG) bearing carboxylate charges and hydrophobic radicals Hy having the following Formula: 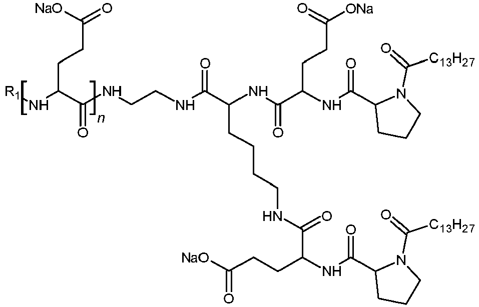 M = 0.02 to 0.1, n = 10 to 50, R₁ = H or pyroglutamate,
with semaglutide.

### EXAMPLES

### Part A - Chemistry

Precursors of Hydrophobic Grafts and Co-polyamino acids bearing Carboxylate Charges and Hydrophobic Grafts could be obtained as described in US2020306379A1 and in WO2019110773.

| **No.** | **CO-POLYAMINO ACIDS BEARING CARBOXYLATE CHARGES AND HYDROPHOBIC RADICALS** |
|---|---|
| A1 | 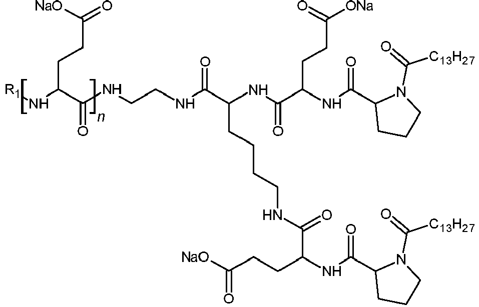 M = 0.05, n = 20 |
| | R₁ = H or pyroglutamate |

In particular Co-polyamino acid A1 corresponds to Co-polyamino acid B22 from US2020306379A1 and WO2019110773.

### Part B - Compositions

### Example B1: Stock Solutions

### Example B1a: Solutions of Caarilintide

Solutions of cagrilintide have been prepared by weighing the adequate amount of cagrilintide powder and adding the desired amount of water to reach the desired concentration. A clear solution was obtained by gentle mixing via vial inversions.

### Example B1b: Solutions of Semaglutide

Solutions of semaglutide have been prepared by weighing the adequate amount of semaglutide powder and adding the desired amount of water to reach the desired concentration. A clear solution was obtained by gentle mixing via vial inversions.

### Example B1c: Solutions of Co-polvamino acid A1

Solutions of co-polyamino acid A1 were prepared by weighing the adequate amount of co-polyaminoacid A1 powder, then dissolving it in water. An adjustement to pH 5.4 using NaOH and HCl 1M was done prior to adding water to reach the desired concentration. A clear solution was obtained.

### Example B2: Compositions of the invention comprising Cagrilintide and Semaglutide

### Example B2a: Compositions of Cagrilintide, Semaglutide and Co-polyamino acid A1

Solutions according to the invention were prepared as follows:
- Addition of concentrated aqueous solutions of excipients (i.e. m-cresol (m-cr), mannitol (man)) to the solution of co-polyaminoacid A1 (PLG-A1).
- pH adjustment of this solution to the desired pH when needed (5.4;5.8)
- addition of semaglutide (Sema) solution
- addition of cagrilintide (Cagri) solution
- final pH adjustment if needed, using NaOH 1N or HCl 1N
- addition of water to reach the final desired volume and concentrations.

A final filtration onto 0.22µm filter was done prior to filling.
The following compositions have been prepared:

| Example | [Sema] (mg/ml) | [Cagri] (mg/ml) | PLG-A1 (mg/ml) | Excipients | pH |
|---|---|---|---|---|---|
| B2a-2 | 3.2 | 3.2 | 15 | man 36 mg/mL | 5.4;5.8 |
| B2a-3 | 3.2 | 3.2 | 15 | man 36 mg/mL, m-cr 35 mmol/L | 5.4;5.8 |

Using these compositions, 3 mL cartridges have been filled with 0.5 mL of formulation and sealed without the presence of air bubbles.

### Part C - Stability

### Example C1: Monitoring stability

### Monitoring of Physical Stability

The cartridges containing the compositions were placed at 30°C. A physical stress was applied to the cartridges in the form of 20 daily container manual inversions (40 inversions on Mondays ans Fridays, none on Saturdays ans Sundays).

At 14 and 26 days, one cartridge per composition was retrieved for physical stability assessment. Physical stability was monitored by visual inspection under USP<790> conditions, as well as by MFI sub-visible particle content measurement.

For MFI measurements, the cartridges were opened, and 100µL were sampled without moving the plunger. These 100µL were added to 900µL of water filtered onto 0.22µm filter to yield 1mL of sample for MFI measurement. Sub-visible particle content per mL was then measured on the diluted sample. Using the dilution factor of 10, the number of sub-visible particles per mL of formulation was then calculated.

### Monitoring of Chemical Stability

For the monitoring of chemical stability, cartridges were placed at 37°C. At 14 and 26 days, one cartridge per composition was retrieved for analysis.

Chemical stability was assessed both by RP-HPLC analysis to monitor each peptide's purity, and by SEC-HPLC to monitor the formation of high molecular weight products (HMWP)

### Example C2: Stability Results

Results of visual inspection and MFI measurements done on day 14 and 26 of accelerated physical stability at 30°C studies are presented below:

| Formulation | Part >5µm (/mL) | | Part>10µm (/mL) | | Part>25µm (/mL) | | Visual inspection | |
|---|---|---|---|---|---|---|---|---|
| | T14D | T26D | T14D | T26D | T14D | T26D | T14D | T26D |
| B2a-2 - 5.4 | 161 | 161 | 46 | 69 | 0 | 46 | clear | clear |
| B2a-2 - 5.8 | 2799 | 1399 | 1055 | 413 | 321 | 183 | clear | clear |
| B2a-3 - 5.4 | 160 | 1054 | 0 | 206 | 0 | 46 | clear | clear |
| B2a-3 - 5.8 | 321 | 390 | 46 | 161 | 0 | 92 | clear | clear |

These formulations appear stable during this accelerated stability study, with visual aspect and sub-visible particle amounts remaining satisfactory.

Results of the chemical stability study are presented below.

| Formulation | Cagrilintide impurity levels (%, delta vs T0) | | Semaglutide impurity levels (%, delta vs T0) | | HMWP content (%, delta vs T0) | |
|---|---|---|---|---|---|---|
| | T14D | T26D | T14D | T26D | T14D | T26D |
| B2a-2 - 5.4 | +1.8 | +2.2 | +1.3 | +2.6 | +0.0 | +0.1 |
| B2a-2 - 5.8 | +1.8 | +2.4 | +1.2 | +2.3 | +0.0 | +0.1 |
| EMO17(15 mg/mL BC, pH5.4 + cresol) | +0.8 | +1.4 | +1.4 | +2.6 | +0.1 | +0.2 |
| B2a-3 - 5.4 | | | | | | |
| B2a-3 - 5.8 | +1.5 | +1.9 | +1.3 | +2.3 | +0.1 | +0.3 |

As can be seen from the table above, the level of impurities after 26 days at 37°C is below 2.5% for cagrilintide and below 3% for semaglutide.

For comparison, Novo's application WO2023/183067 disclosed a higher level of impurity increase at 37°C at 28 days for cagrilintide and for semaglutide.

## Claims

1. A composition, in the form of an aqueous injectable solution, whose pH is comprised from 5.0 to 6.0, comprising:
- semaglutide
- cagrilintide, and
- a co-polyamino acid (PLG) bearing carboxylate charges and hydrophobic radicals Hy, said co-polyamino acid consisting of glutamic or aspartic units, and said hydrophobic radicals Hy being according to formula X below: 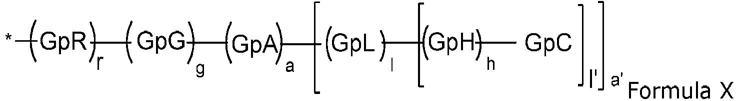 in which
- GpR is chosen from the radicals according to formulas VII, VII' or VII': 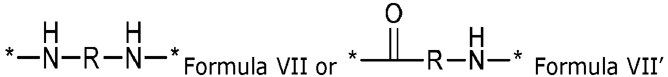 or 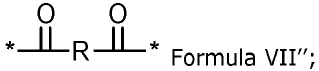
- identical or different GpG and GpH are chosen from the radicals according to formulas XI or XI': 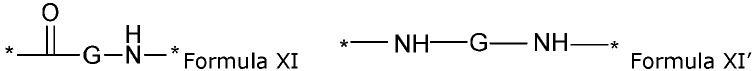
- GpA is chosen from the radicals according to formula VIII: 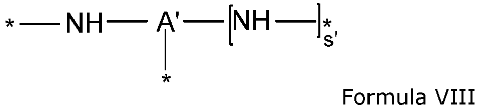 in which A' is chosen from the radicals according to formula VIII', VIII" or VIII‴: 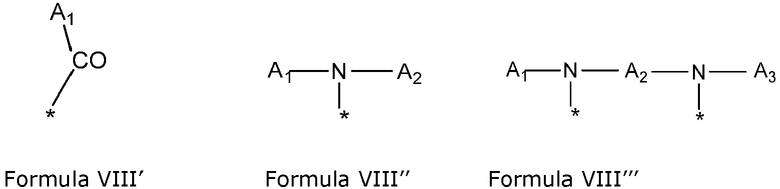
- GpL is chosen from the radicals according to formula XII: 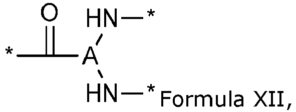
- GpC is a radical according to formula IX: 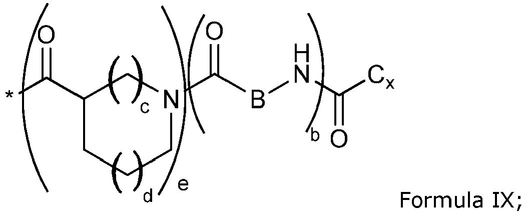 the * indicates the attachment sites of the different groups bonded by the amide functions;
- a is an integer equal to 0 or to 1 and a' = 1 if a = 0 and a' = 1, 2 or 3 if a = 1;
- a' is an integer equal to 1, to 2, or to 3
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- l is an integer equal to 0 or 1 and I' = 1 if I = 0, and I' = 2 if l = 1;
- r is an integer equal to 0, to 1, or to 2, and
- s' is an integer equal to 0 or 1, and
- if e is different from 0, then at least one of g, h or l is different from 0;
- A, A₁, A₂ and A₃, identical or different, are linear or branched alkyl radicals, and/or substituted by a radical derived from a saturated, unsaturated or aromatic ring, comprising 1 to 8 carbon atoms.
- B is a linear or branched alkyl radical and/or comprising an aromatic nucleus, comprising 1 to 9 carbon atoms or an unsubstituted ether or polyether radical comprising from 4 to 14 carbon atoms and from 1 to 5 oxygen atoms;
- Cₓ is a linear or branched monovalent alkyl radical, and/or comprising a cyclic part, in which x indicates the number of carbon atoms and:
▪ when the hydrophobic radical -Hy carries 1 -GpC, then 9 ≤ x ≤ 25,
▪ when the hydrophobic radical -Hy carries 2 -GpC, then 9 ≤ x ≤ 15,
▪ when the hydrophobic radical -Hy carries 3 -GpC, then 7 ≤ x ≤ 13,
▪ when the hydrophobic radical -Hy carries 4 -GpC, then 7 ≤ x ≤ 11,
▪ when the hydrophobic radical -Hy carries at least 5 -GpC, then 6 ≤ x ≤ 11,
- G is a branched alkyl radical of 1 to 8 carbon atoms, with said alkyl radical bearing one or more free carboxylic acid functions,
- R is a radical chosen from the group consisting of a divalent alkyl radical, linear or branched, comprising 1 to 12 carbon atoms, a divalent alkyl radical, linear or branched, comprising 1 to 12 carbon atoms bearing one or more -CONH₂ functions, or a unsubstituted ether or polyether radical comprising from 4 to 14 carbon atoms and 1 to 5 oxygen atoms:
- the hydrophobic radical(s) -Hy according to formula X being bonded to the PLG:
- via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
- via a covalent bond between a nitrogen atom of the hydrophobic radical -Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0 < M ≤ 0.5;
- when several hydrophobic radicals are borne by a co-polyamino acid, then they are identical or different,
- the degree of polymerization DP in glutamic or aspartic units for PLG chains is comprised from 5 to 250;
- free carboxylic acid functions being in the form of an alkali metal salt chosen from the group consisting of Na⁺ and K⁺.

2. A composition according to Claim 1, **characterized in that** the semaglutide is at a concentration of about 0.25 mg/ml to about 22 mg/ml.

3. A composition according Claim 1 or 2, **characterized in that** it comprises cagrilintide at a concentration of about 0.25 mg/ml to about 22 mg/ml.

4. A composition according any one of Claims 1 to 3, **characterized in that** the co-polyamino acid bearing carboxylate charges and hydrophobic radicals is chosen from the co-polyamino acids according to formula XXXa' below: 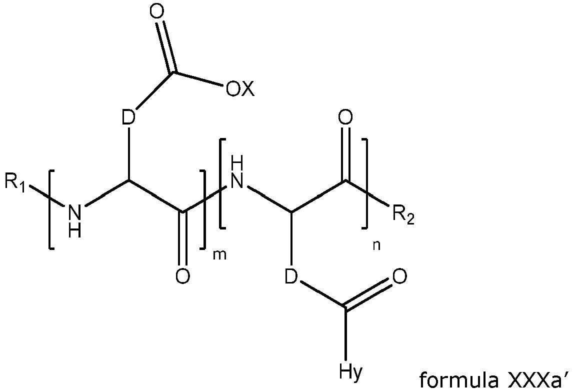 in which
- D represents, independently, either a -CH₂- group (aspartic unit) or a -CH₂-CH₂-group (glutamic unit),
- Hy is a hydrophobic radical chosen from the hydrophobic radicals according to formula X, in which r=1 and GpR is a radical according to formula VII,
- R₁ is a hydrophobic radical chosen from the hydrophobic radicals according to formula X in which r=0 or r=1 and GpR is a radical according to formula VII', or a radical chosen from the group consisting of an H, a linear acyl group in C2 to C10, a branched acyl group in C4 to C10, a benzoyl, a terminal "amino acid" unit and a pyroglutamate,
- R₂ is a hydrophobic radical chosen from the hydrophobic radicals according to formula X in which r=1 and GpR is a radical according to formula VII, or a -NR'R" radical, R' and R", either identical or different, being chosen from the group comprised by H, linear or branched or cyclic alkyls in C2 to C10, or benzyl, and said R' and R" alkyls which may form together one or more carbonated, saturated, unsaturated and/or aromatic cycle(s), and/or may comprise hetero-atoms, chosen from the group comprised of O, N and S;
- X represents a cationic entity chosen from the group comprising alkaline cations;
- n + m represents the degree of polymerization DP of the co-polyamino acid, namely, the average number of monomeric units per co-polyamino acid chain and 5 ≤ n + m ≤ 250.

5. A composition according any one of Claims 1 to 4, **characterized in that** the co-polyamino acid bearing carboxylate charges and hydrophobic radicals is chosen from the co-polyamino acids according to formula XXXa' in which n = 0 according to formula XXXb below: 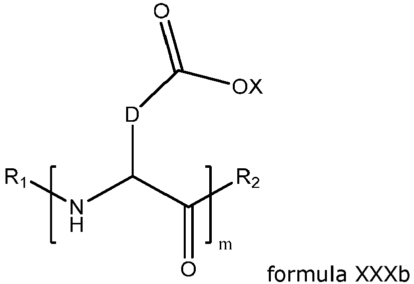 in which m, X, D, R₁ and R₂ have the definitions given in Claim 8 and at least R₁ or R₂ is hydrophobic radical according to formula X.

6. The composition according to according any one of Claims 1 to 5, **characterized in that** the co-polyamino acid bearing carboxylate charges and hydrophobic radicals is chosen from the co-polyamino acids according to formula XXXb in which R₂ is a hydrophobic radical according to formula X in which r = 1 and GpR is according to formula VII.

7. The composition according to any of claims 1 to 6, **characterized in that** concentration of co-polyamino acid bearing carboxylate charges and hydrophobic radicals is at most 60 mg/ml.

8. The composition according to any of claims 1 to 7, **characterized in that** it further comprises a preservative.

9. The composition according to any of claims 1 to 8, **characterized in that** said composition is in the form of an aqueous injectable solution, whose pH is comprised from 5.0 to 6.0, comprising
- semaglutide,
- cagrilintide, and
- a co-polyamino acid (PLG) bearing carboxylate charges and hydrophobic radicals Hy having the following Formula: 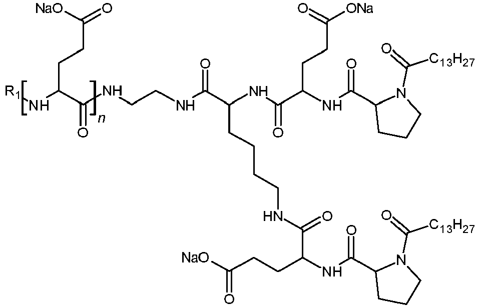 M = 0.02 to 0.1, n = 10 to 50, R₁ = H or pyroglutamate.

10. The composition according to any of claims 1 to 9, for use as a medicament.

11. The composition according to any of claims 1 to 10, for use for treatment of overweight, obesity, diabetes, cardio-vascular disease(s), non-alcoholic steatohepatitis (NASH), and/or cognitive impairment, such as caused by Alzheimer Disease (AD).

12. Injection device comprising a composition such as disclosed in any one of the claims 1 to 11.
